# EUROPEAN PATENT SPECIFICATION

(11) **EP 0 914 142 B1**
(45) Date of publication and mention of the grant of the patent: **08.06.2011**
(21) Application number: 97914975.4
(22) Date of filing: 10.03.1997
(51) Int. Cl.: A61K 38/03, A61K 39/002, A61K 39/02, A61K 39/12

(54) **PEPTIDES WITH INCREASED BINDING AFFINITY FOR AT LEAST THREE HLA-A3-LIKE MOLECULES**
PEPTIDE MIT ERHÖHTER BINDUNGSAFFINITÄT FÜR MINDESTENS DREI HLA-A3-ÄHNLICHE MOLEKÜLE
PEPTIDES PRESENTANT UNE AFFINITE AUGMENTE POUR AU MOINS TROIS MOLECULES DE TYPE HLA-A3

(30) Priority: 11.03.1996 US 13113 P
(43) Date of publication of application: 12.05.1999
(73) Proprietor: Epimmune Inc., San Diego, CA 92121 (US)
(72) Inventor: SETTE, Alessandro, La Jolla, CA 92037 (US); CHESNUT, Robert, W., Cardiff, CA 92007 (US); SIDNEY, John, San Diego, CA 92130-2102 (US)
(74) Representative: Inspicos A/S
(86) International application number: PCT/US1997/003778
(87) International publication number: WO 1997/033602

(56) References cited:
- EP-A1- 0 728 764
- WO-A-94/03205
- WO-A1-94/03205
- WO-A1-95/03777
- WO-A1-95/11255
- US-A- 5 200 320
- US-A1- 2005 271 676
- KAST W M ET AL: "ROLE OF HLA-A MOTIFS IN IDENTIFICATION OF POTENTIAL CTL EPITOPES INHUMAN PAPILLOMAVIRUS TYPE 16 E6 AND E7 PROTEINS" JOURNAL OF IMMUNOLOGY, THE WILLIAMS AND WILKINS CO. BALTIMORE, US, vol. 152, no. 8, 1994, pages 3904-3912, XP002936659 ISSN: 0022-1767
- VITIELLO ANTONELLA ET AL: "Development of a lipopeptide-based therapeutic vaccine to treat chronic HBV infection: I. Induction of a primary cytotoxic T lymphocyte response in humans" JOURNAL OF CLINICAL INVESTIGATION, vol. 95, no. 1, 1995, pages 341-349, XP000923044 ISSN: 0021-9738
- DONNELLY J J ET AL: "IMMUNIZATION WITH DNA" JOURNAL OF IMMUNOLOGICAL METHODS, ELSEVIER SCIENCE PUBLISHERS B.V.,AMSTERDAM, NL, vol. 176, no. 2, 21 December 1994 (1994-12-21), pages 145-152, XP001005665 ISSN: 0022-1759
- SIDNEY ET AL.: 'Definition of an HLA-A3-Like Supermotif Demonstrates the Overlapping Peptide-Binding Repertoires of Common HLA Molecules' HUMAN IMMUNOLOGY vol. 45, 1996, pages 79 - 93, XP002055569
- SIDNEY ET AL.: 'Practical, Biochemical and Evolutionary Implications of the Discovery of HLA Class I Supermotifs' IMMUNOLOGY TODAY vol. 17, no. 6, June 1996, pages 261 - 266, XP004034609
- KUBO ET AL.: 'Definition of Specific Peptide Motifs for Four Major HLA-A Alleles' JOURNAL OF IMMUNOLOGY vol. 152, no. 8, 1994, pages 3913 - 3924, XP000566323
- KAST ET AL.: 'Role of HLA-A Motifs in Identification of Potential CTL Epitopes in Human Papillomavirus Type 16 E6 and E7 Proteins' JOURNAL OF IMMUNOLOGY vol. 152, no. 8, 1994, pages 3904 - 3912, XP002936659
- RUPPERT ET AL.: 'Class I MHC-peptide Interaction: Structural and Functional Aspects' BEHRING INSTITUTE MITTEILUNGEN no. 94, 1994, pages 48 - 60, XP000604845
- RAMMENSEE ET AL.: 'MHC Ligands and Peptide Motifs: First Listing' IMMUNOGENETICS vol. 41, no. 4, 1995, pages 178 - 228, XP000673045
- ENGELHARD: 'Structure of Peptides Associated With MHC Class I Molecules' CURRENT OPINION IN IMMUNOLOGY vol. 6, 1994, pages 13 - 23, XP002912059
- KUMAR ET AL.: 'Amino Acid Variations at a Single Residue in an Autoimmune Peptide Profoundly Affect its Properties: T-cell Activation, Major Histocompatibility Complex Binding and Ability to Block Experimental Allergic Encephalomyelitis' PROC. NATL. ACAD. SCI. U.S.A. vol. 87, February 1990, pages 1337 - 1341, XP000103572
- ALEXANDER J. ET AL: 'Derivation of HLA-A11/Kb transgenic mice: functional CTL repertoire and recognition of human A11-restricted CTL epitopes.' JOURNAL OF IMMUNOLOGY (BALTIMORE, MD. : 1950) 15 NOV 1997 vol. 159, no. 10, 15 November 1997, pages 4753 - 4761 ISSN: 0022-1767
- VAN DER BURG S.H. ET AL: 'An HLA class I peptide-binding assay based on competition for binding to class I molecules on intact human B cells. Identification of conserved HIV-1 polymerase peptides binding to HLA-A*0301.' HUMAN IMMUNOLOGY DEC 1995 vol. 44, no. 4, December 1995, pages 189 - 198 ISSN: 0198-8859
- CELIS E. ET AL: 'Identification of potential CTL epitopes of tumor-associated antigen MAGE-1 for five common HLA-A alleles' MOLECULAR IMMUNOLOGY, PERGAMON, GB vol. 31, no. 18, 01 December 1994, pages 1423 - 1430, XP023681890 ISSN: 0161-5890
- INTERNET CITATION: 'MHC Binding Assays for SLFRAVITK', [Online] Retrieved from the Internet: <URL:http://www.immuneepitope.org/mhc_list. php?structureCategory=&total_rows=17&assay[ ]=bcell&assay[]=tcell&assay[]=mhc&assay[]=e lution&response=Positive&queryType=true&lin earSequence=SLFRAVITK> [retrieved on 2009-12-14]
- SETTE A. ET AL: 'Peptide binding to the most frequent HLA-A class I alleles measured by quantitative molecular binding assays' MOLECULAR IMMUNOLOGY, PERGAMON, GB vol. 31, no. 11, 01 August 1994, pages 813 - 822, XP023786376 ISSN: 0161-5890
- GAVIOLI R. ET AL: 'Effect of anchor residue modifications on the stability of HLA-A11/peptide complexes.' BIOCHEMICAL AND BIOPHYSICAL RESEARCH COMMUNICATIONS 5 JAN 1995 vol. 206, no. 1, 05 January 1995, pages 8 - 14 ISSN: 0006-291X
- INTERNET CITATION: 'Restricting MHC Allele for ASFDKAKLK', [Online] Retrieved from the Internet: <URL:http://www.immuneepitope.org/mhc_allel e_list.php?structureCategory=&total_rows=7& assay[]=bcell&assay[]=tcell&assay[]=mhc&ass ay[]=elution&response=&queryType=true&linea rSequence=ASFDKAKLK>
- INTERNET CITATION: 'MHC bining assays for AVFIHNFKR', [Online] Retrieved from the Internet: <URL:http://www.immuneepitope.org/mhc_list. php?structureCategory=&total_rows=13&assay[ ]=bcell&assay[]=tcell&assay[]=mhc&assay[]=e lution&response=Positive&queryType=true&lin earSequence=AVFIHNFKR>
- PIRISI M. ET AL: 'Reactivity to B cell epitopes within hepatitis C virus core protein and hepatocellular carcinoma.', 01 January 1995, CANCER RESEARCH 1 JAN 1995, VOL. 55, NR. 1, PAGE(S) 111 - 114, ISSN 0008-5472
- INTERNET CITATION: 'MHC Binding Assays for KTSERSQPR', [Online] Retrieved from the Internet: <URL:http://www.immuneepitope.org/mhc_list. php?structureCategory=&total_rows=12&assay[ ]=bcell&assay[]=tcell&assay[]=mhc&assay[]=e lution&response=Positive&queryType=true&lin earSequence=KTSERSQPR>
- INTERNET CITATION: 'MHC binding assays for peptide QLFTFSPRR', [Online] Retrieved from the Internet: <URL:http://www.immuneepitope.org/mhc_list. php?structureCategory=&total_rows=10&assay[ ]=bcell&assay[]=tcell&assay[]=mhc&assay[]=e lution&response=Positive&queryType=true&lin earSequence=QLFTFSPRR>
- CHING W.-M. ET AL: 'INTERACTION OF IMMUNE SERA WITH SYNTHETIC PEPTIDES CORRESPONDING TO THE STRUCTURAL PROTEIN REGION OF HEPATITIS C VIRUS' PROCEEDINGS OF THE NATIONAL ACADEMY OF SCIENCES OF USA, NATIONAL ACADEMY OF SCIENCE, WASHINGTON, DC, US vol. 89, 01 April 1992, pages 3190 - 3194, XP002048541 ISSN: 0027-8424
- INTERNET CITATION: 'T cell response assays for peptide QLFTFSPRR', [Online] Retrieved from the Internet: <URL:http://www.immuneepitope.org/tcell_lis t.php?structureCategory=&total_rows=7&assay []=bcell&assay[]=tcell&assay[]=mhc&assay[]= elution&response=Positive&queryType=true&li nearSequence=QLFTFSPRR>
- INTERNET CITATION: 'T Cell Response Assays for peptide KTSERSQPR', [Online] Retrieved from the Internet: <URL:http://www.immuneepitope.org/tcell_lis t.php?structureCategory=&total_rows=25&assa y[]=bcell&assay[]=tcell&assay[]=mhc&assay[] =elution&response=Positive&queryType=true&l inearSequence=KTSERSQPR>

## Description

### BACKGROUND OF THE INVENTION

The present invention relates to compositions and methods for preventing, treating or diagnosing a number of pathological states. In particular, it provides novel peptides capable of binding selected major histocompatibility complex (MHC) molecules and inducing an immune response.

MHC molecules are classified as either Class I or Class II molecules. Class II MHC molecules are expressed primarily on cells involved in initiating and sustaining immune responses, such as T lymphocytes, B lymphocytes, macrophages, and the like. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral, fungal, and parasitic infections.

The relationship between binding affinity for MHC class I molecules and immunogenicity of discrete peptide epitopes has been analyzed in two different experimental approaches (Sette, et al., J. Immunol., 153:5586-5592 (1994)). In the first approach, the immunogenicity of potential epitopes ranging in MHC binding affinity over a 10,000-fold range was analyzed in HLA-A*0201 transgenic mice. In the second approach, the antigenicity of approximately 100 different hepatitis B virus (HBV)-derived potential epitopes, all carrying A*0201 binding motifs, was assessed by using PBL of acute hepatitis patients. In both cases, it was found that an affinity threshold of approximately 500 nM (preferably 500 nM or less) determines the capacity of a peptide epitope to elicit a CTL response. These data correlate well with class I binding affinity measurements of either naturally processed peptides or previously described T cell epitopes. These data indicate the important role of determinant selection in the shaping of T cell responses.

In general, CTL responses are not directed against all possible epitopes. Rather, they are restricted to a few immunodominant determinants (Zinkernagel, et al., Adv. Immunol. 27, 51-59 (1979); Bennink, et al., J. Exp. Med. 168, 1935-1939 (1988); Rawle, et al., J. Immunol. 146, 3977-3984 (1991)). It has long been recognized that immunodominance (Benacerraf, et al., Science. 175, 273-279 (1972)) could be explained by either the ability of a given epitope to selectively bind a particular MHC molecule (determinant selection theory) (Vitiello, et al., J. Immunol. 131:1635 (1983); Rosenthal, et al., Nature 267, 156-158 (1977)) or being selectively recognized by the existing TCR specificity (repertoire theory) (Klein, J., Immunology, the Science of Self-Nonself Discrimination, John Wiley & Sons, New York, pp. 270-310 (1982)). It has been demonstrated that additional factors, mostly linked to professing events, can also play a key role in dictating, beyond strict immunogenicity, which of the many potential determinants will be presented as immunodominant (Sercarz, et al., Annu. Rev. Immunol. 11, 729-766 (1999)).

The ability to modulate the binding affinity of a particular immunogenic peptide for one or more HLA molecules and thereby modulate the immune response elicited by the peptide would greatly enhance the usefulness of peptide-based vaccines and therapeutic agents. The present invention provides these and other advantages.

WO 94/03205 discloses peptide compositions capable of specifically binding selected MHC alleles and inducing T cell activation in T cells restricted by the MHC allele. The peptides are useful to elicit an immune response against a desired antigen.

In Rammensee et al, Immunogenetics (1995) 41: 178-228, entitled "MHC ligands and peptide motifs: first listing" a compendium of MHC peptide motifs and MHC ligands are provided.

Deckhut et al., J. of Virology, Jan 1992, p.440-447 discloses that SV40 TAA expressed in H-2b SV40-transformed cells induces the generation of Lyt-2+ (CD8+) CTLs, which are involved in tumour rejection, in syngeneic mice. The minimal and optimal residues of T antigen recognized by four CTL clones is determined by using synthetic peptides.

In the "Definition of Specific Petptide Motifs for Four Major HLA-A Alleles", J. Immunology, Vol. 152, No. 8, 15 April 1994, pp 3913-24, Kubo et al., discusses and characterises allele-specific motifs for the Human MHC class I molecules, HLA-A1, A3, A11 and A24.

### SUMMARY OF THE INVENTION

The scope of the present invention is defined by the claims and any information that does not fall within the claims is provided for information only.

There are disclosed herein peptides and nucleic acids encoding them for use in vaccines and therapeutics. Also disclosed are methods of inducing a cytotoxic T cell response against a preselected antigen in a patient, the method comprising contacting a cytotoxic T cell with an immunogenic peptide of the invention. The peptides of the invention may be derived from a number of antigens including viral antigens, tumour associated antigens, parasitic antigens, fungal antigens and the like. The method disclosed herein can be carried out *in vitro* and *in vivo*. Preferably the peptides are contacted with the cytotoxic T cell by administering to the patient a nucleic acid molecule comprising a sequence encoding the immunogenic peptide.

The present disclosure provides a composition comprising an immunogenic peptide of between 9 and 15 residues which binds to at least two HLA-A3-like molecules selected from the group comprising A*0301, A*1101, A*3101, A*3301 and A*6801 with a dissociation constant of less than 500nM and induces a cytotoxic T cell response, which immunogenic peptide comprises an A3 supermotif consisting of a sequence of 9 residues from the N-terminus to the C-terminus as follows:
a first primary anchor residue at the second position selected from the group consisting of A, L, I, V, M, S and T and a second primary anchor residue at the ninth position selected from the group consisting of R and K; and
one or more secondary anchor residues selected from the group consisting of (i) Y F, or W, at the third position, (ii) Y, F, or W at the sixth position, (iii) Y, F, or W at the seventh position, (iv), P at the eighth position, and any combination of (i), (ii), (iii) and (iv).

The present invention provides a method of identifying peptides that bind to an HLA-A3 like molecule with a dissociation constant of less than 500 nM, the method comprising the following steps:
screening an amino acid sequence of an antigenic protein for the presence of the binding motif of claim 1; selecting one or more subsequences in the antigenic protein having the binding motif; preparing test peptides of 8 and 11 residues comprising the selected subsequences; determining the ability of the test peptides to bind to the HLA-A3 like molecule; and identifying peptides with a dissociation constant of less than 500
nM.

### Definitions

The term "peptide" is used interchangeably with "oligopeptide" in the present specification to designate a series of residues, typically L-amino acids, connected one to the other typically by peptide bonds between the alpha-amino and carbonyl groups of adjacent amino acids. The oligopeptides of the invention are less than about 15 residues in length and usually consist of between about 8 and about 11 residues, preferably 9 or 10 residues.

An "immunogenic peptide" is a peptide which comprises an allele-specific motif or super-motif such that the peptide will bind an MHC molecule and induce a CTL response. Immunogenic peptides of the invention are capable of binding to an appropriate HLA-A3-like molecule and inducing a cytotoxic T cell response against the antigen from which the immunogenic peptide is derived.

A "primary anchor residue" is an amino acid at a specific position along a peptide sequence which may provide a contact point between the immunogenic peptide and the MHC molecule. One to three, usually two, primary anchor residues within a peptide of defined length generally defines a motif for an immunogenic peptide. These residues are typically in close contact with the peptide binding groove, with their side chains buried in specific pockets of the groove itself. Typically, the primary anchor residues are located in the 2 and 9 position of 9 residue peptide.

A "secondary anchor residue" is an amino acid which occurs in a significantly higher frequency than would be expected by random distribution at a particular position other than the primary anchors in a peptide. Alternatively, secondary anchors can be identified as those with a higher frequency among high affinity binding peptides or are associated with high affinity binding. Presence or absence of particular residues in these positions can be used to finely modulate the binding affinity of a peptide comprising a particular motif.

As used herein, "negative binding residues" are amino acids which if present at certain positions (typically not primary anchor positions) will result in decreased binding affinity for the target HLA molecule.

The term "motif" refers to the pattern of residues in a peptide of defined length, usually about 8 to about 15 amino acids, which is recognized by a particular MHC molecule. The peptide motifs are typically different for each human MHC allele and differ in the pattern of the primary and secondary anchor residues.

A "supermotif" refers to motifs that, when present in an immunogenic peptide, allow the peptide to bind more than one HLA antigen. The supermotif preferably is recognized with high or intermediate affinity (as defined below) by at least one HLA allele, preferably recognized by at least two alleles, more preferably recognized by at least three alleles, and most preferably recognized by more than three alleles.

HLA class I molecules that share somewhat similar peptide-binding motifs are grouped into HLA supertypes. An "HLA supertype or family", as used herein, describes sets of molecules grouped on the basis of shared peptide-binding specificities, rather than serologic supertypes based on shared antigenic determinants.

An "HLA-A3-like" HLA molecule (also referred to as an allele) as used herein refers to a group of HLA molecules encoded by HLA-A alleles that share an overlapping peptide binding motif with the HLA-A3 supermotif disclosed here. The 9 residue supermotif shared by these alleles comprises the following primary anchor residues: A, L, I, V, M, S, or, T at position 2 and positively charged residues, such as R and K at position 9 (the C-terminus in 9-mers). Exemplary members of this family, identified by either serology or DNA typing, include: A3 (A*0301), A11 (A*1101), A31 (A*3101), A*3301, and A*6801. Other members of the family include A34, A66, and A*7401. As explained in detail below, binding to each of the individual alleles can be finely modulated by substitutions at the secondary anchor positions.

The "HLA-A2-like" supertype is characterized by a preference for peptide ligands with small or aliphatic amino acids (L, I, V, M, A, and T) at position 2 and the C-terminus. The family is comprised of at least eight HLA-A alleles (A*0201, A*0202, A*0203, A*0204, A*0205, A*0206, A*6802, and A*6901).

The "HLA-B7-like" supertype is comprised of products from at least a dozen HLA-B alleles (B7, B*3501-3, B51, B*5301, B*5401, B*5501, B*5502, B*5601, BB*6701, and B*7801) (Sidney, et al., J Immunol 154:247 (1995); Barber, et al., Curr Biol 5:179 (1995); Hill, et al, Nature 360:434 (1992); Rammensee, et al., Immunogenetics 41:178 (1995)), and is characterized by molecules that recognize peptides bearing proline in position 2 and hydrophobic or aliphatic amino acids (L, I, V, M, A, F, W, and Y) at their C-terminus.

The phrases "isolated" or "biologically pure" refer to material which is substantially or essentially free from components which normally accompany it as found in its native state. Thus, the peptides do not contain materials normally associated with their *in situ* environment, e.g., MHC I molecules on antigen presenting cells. Even where a protein has been isolated to a homogenous or dominant band, there are trace contaminants in the range of 5-10% of native protein which co-purify with the desired protein. Isolated peptides of this disclosure do not contain such endogenous co-purified protein.

The term "residue" refers to an amino acid or amino acid mimetic incorporated in a oligopeptide by an amide bond or amide bond mimetic.

### BRIEF DESCRIPTION OF THE DRAWINGS

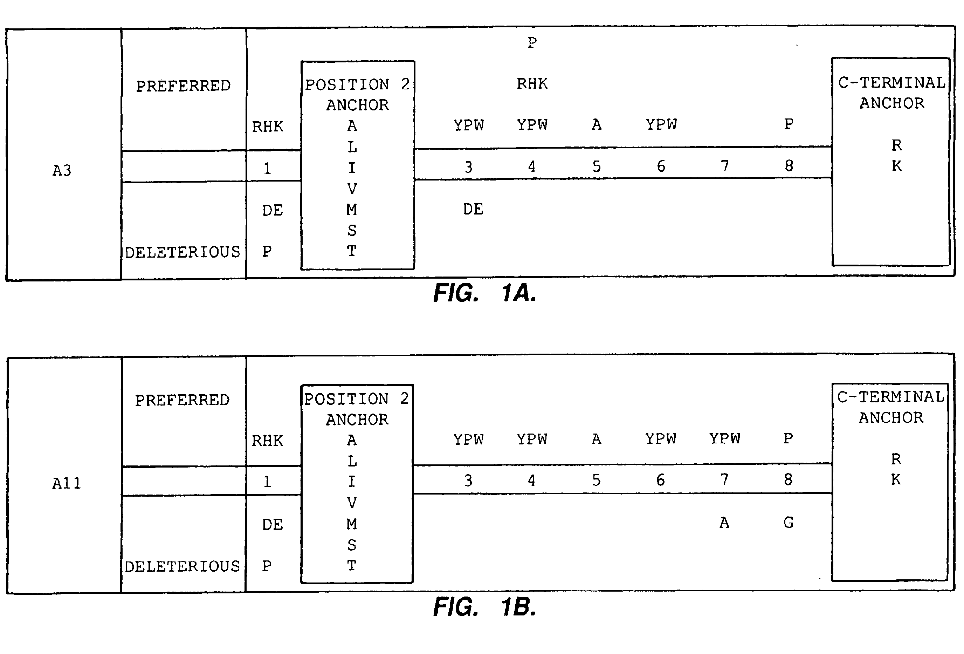

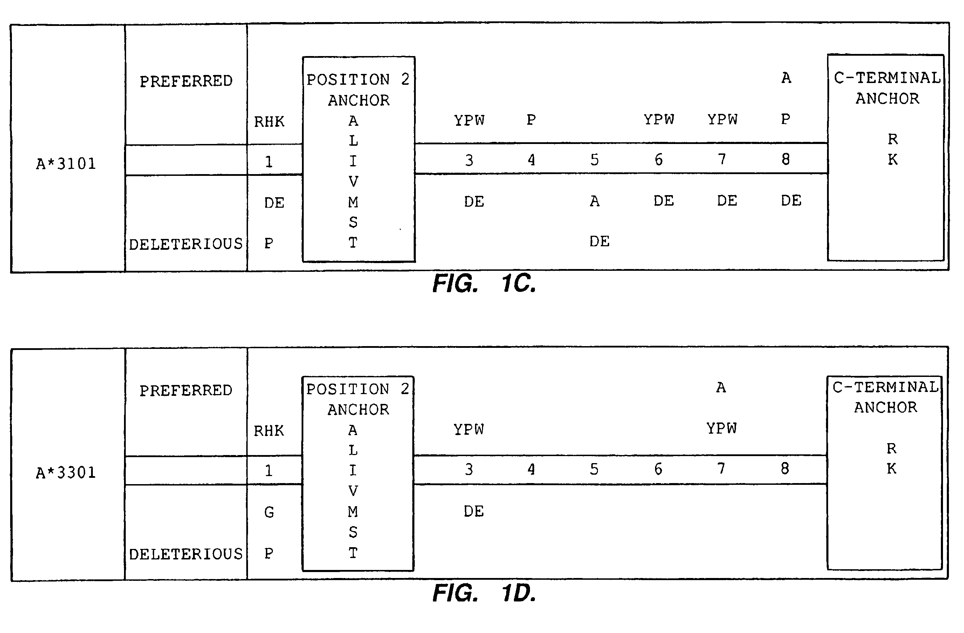

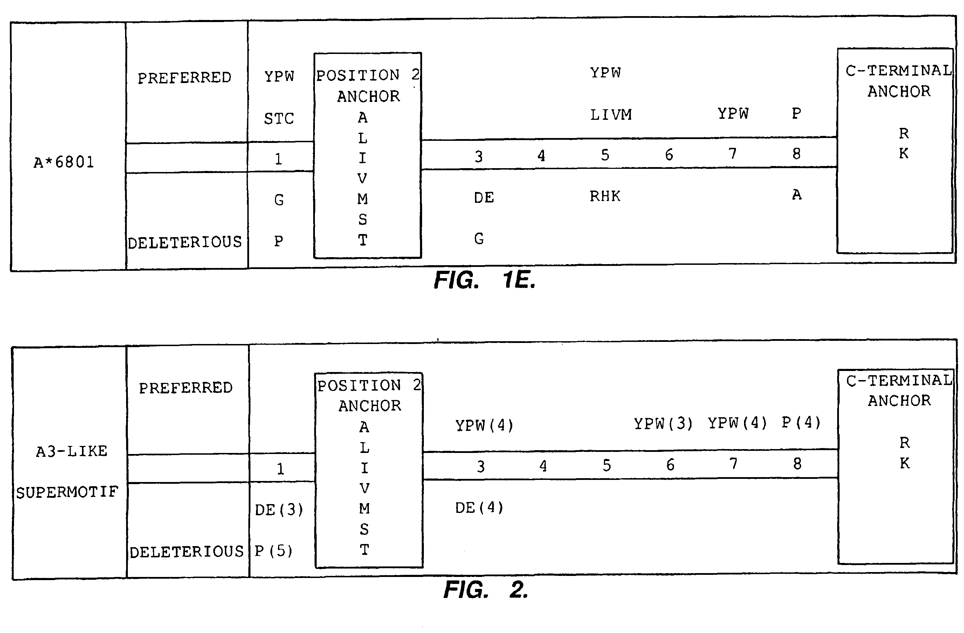

Figure 1 shows refined allele specific motifs of the five A3-like alleles: A*0301, A*1101, A*3101, A*3301, and A*6801. Individual residues, or groups of residues of similar chemical nature, associated for each non-anchor position with either good or poor binding capacities to each individual allele are shown.
Figure 2 shows the A3-like supermotif. Numbers in parenthesis indicate the number of molecules for which the residue or residue group was preferred or deleterious.

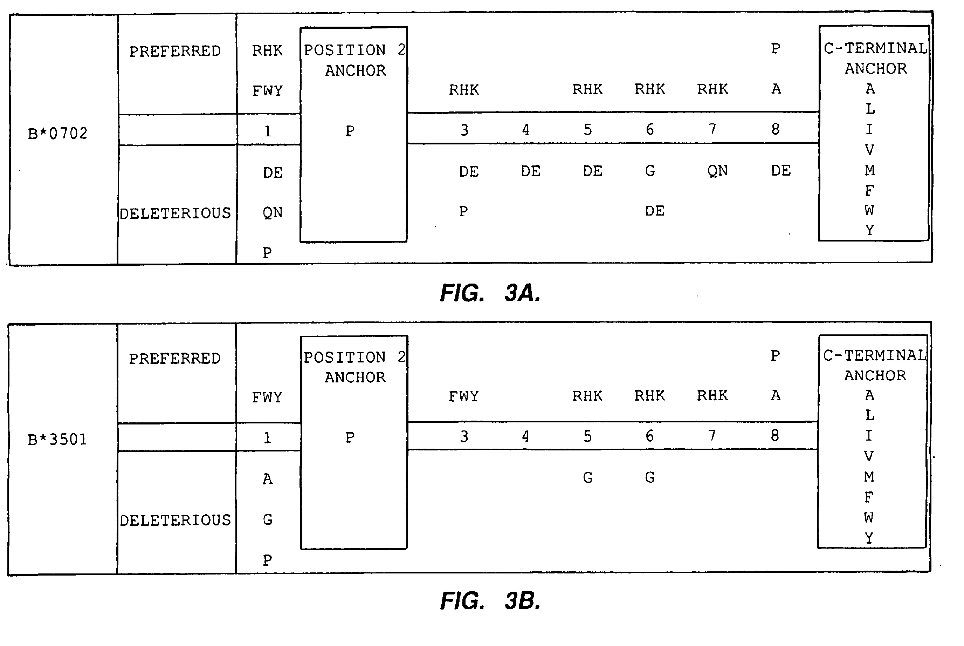

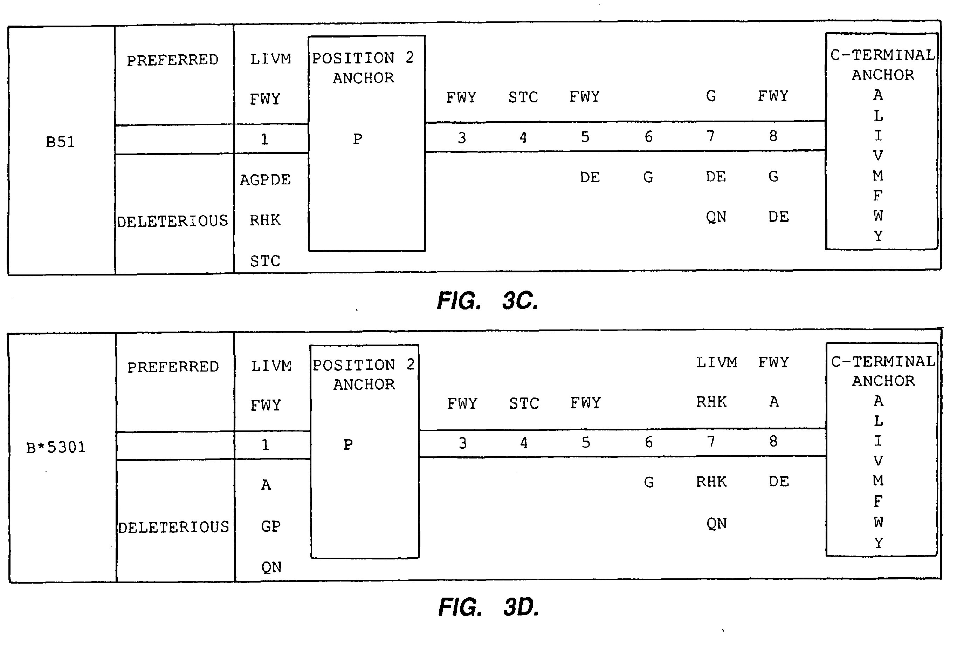

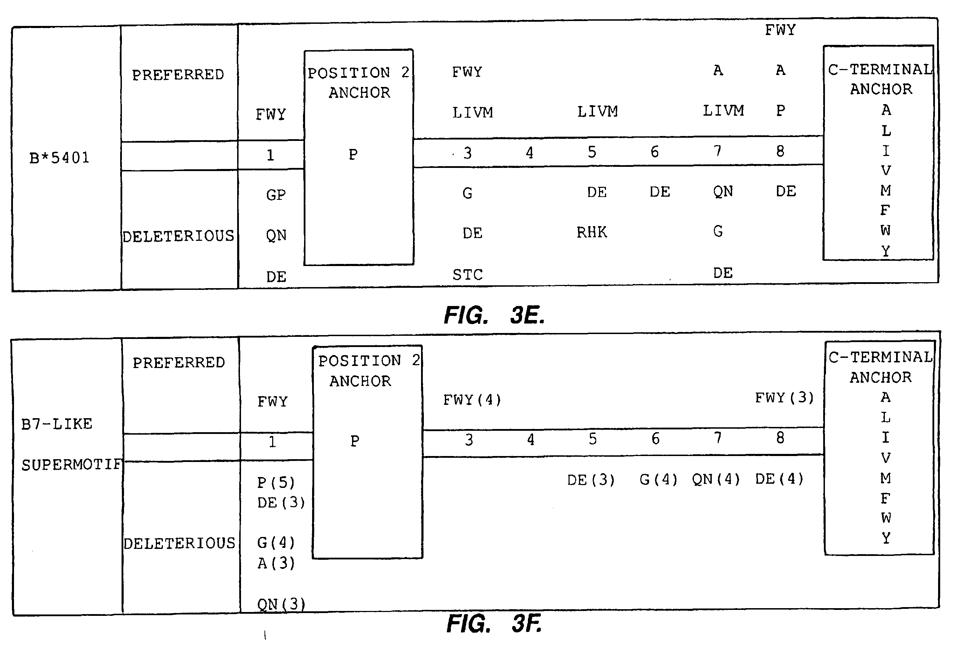

Figure 3 summarizes secondary effects influencing the binding capacity of peptides for a) B*0702, b) B*3501, c) B51, d) B*5301, and e) B*5401. These maps were subsequently used to define the B7-like supermotif (f). Values in parenthesis indicate the frequency that a residue or residue group was preferred or deleterious.

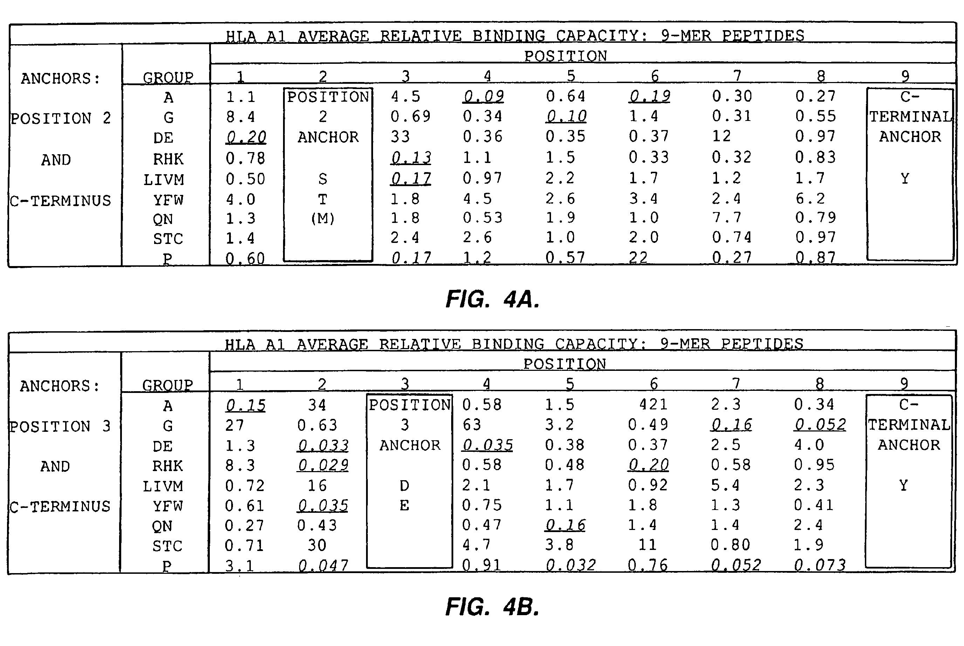

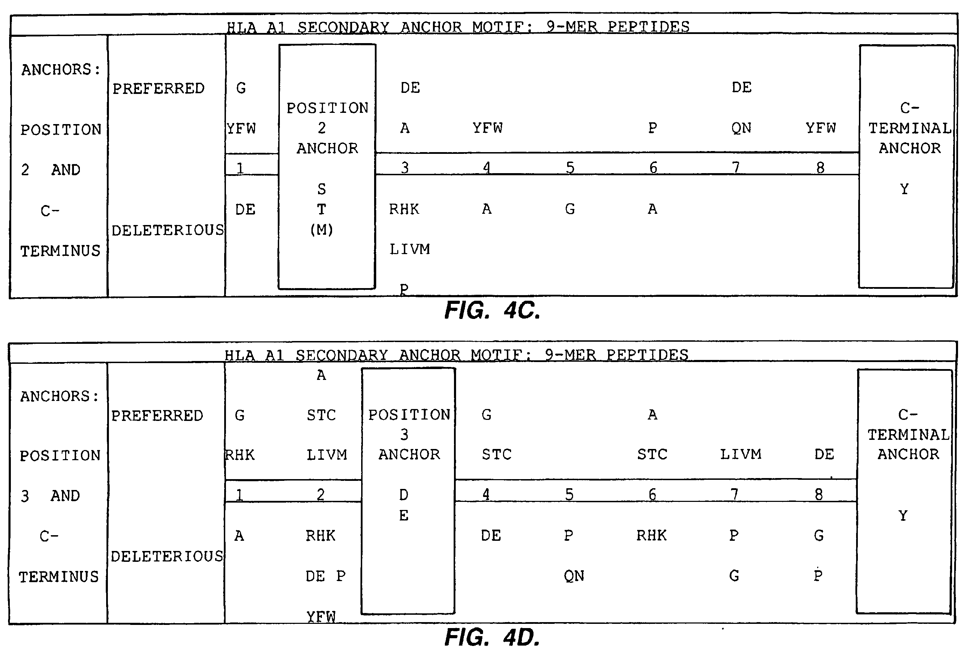

Figure 4 shows relative average binding capacity of the A*0101 submotif 9 mer peptides as function of the different residues occurring at each of the non-anchor positions. Data sets from either 2-9 mer (a) 3-9 mer (b) submotif peptide sets were analyzed and tabulated as described in the Material and Methods. The 2-9 and 3-9 sets contained 101 and 85 different peptides, respectively. Maps of secondary effects influencing the binding capacity of 9-mer peptides carrying the 2-9 (c) 3-9 mer (d) A*0101 submotifs are also shown.

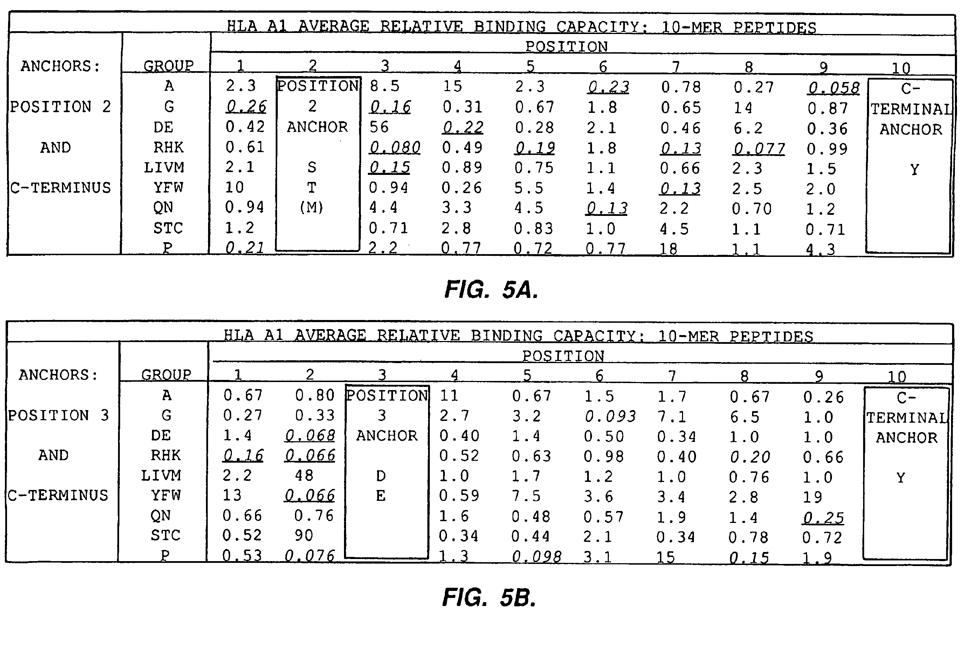

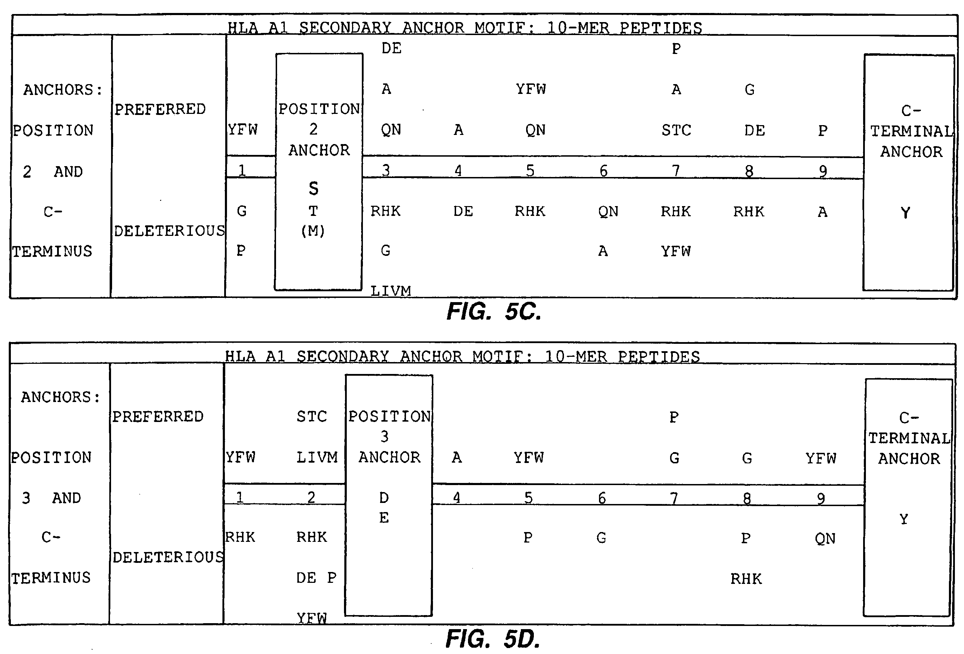

Figure 5 shows relative average binding capacity of the A*0101 submotif 10 mer peptides as function of the different residues occurring at each of the non-anchor positions. Data sets from either 2-10 mer (a) or 3-10 (b) submotif sets of peptides were analyzed and tabulated. The 2-10 and 3-10 sets contained 91 and 89 different peptides, respectively. Maps of secondary effects influencing the binding capacity of 10 mer peptides carrying the 2-10 (c) mer and (1) and or 3-10 mer (d) A1 submotifs are also shown.

### DETAILED DESCRIPTION

The scope of the present invention is defined by the claims and any information that does not fall within the claims is provided for information only. The present invention relates to the determination of allele-specific peptide motifs and supermotifs for human Class I MHC (referred to as HLA) alleles, in particular, HLA-A3-like alleles. These motifs are then used to prepare and modify T cell epitopes from any desired antigen, particularly those associated with human viral diseases, parasitic diseases, fungal diseases, or cancers.

As noted above, high HLA binding affinity is correlated with higher immunogenicity. Higher immunogenicity can be manifested in several different ways. For instance, a higher binding peptide will be immunogenic more often. Close to 90% of high binding peptides are immunogenic, as contrasted with about 50% of the peptides which bind with intermediate affinity. A higher binding peptide will also lead to a more vigorous response. As a result, less peptide is required to elicit a similar biological effect. High binding epitopes are particularly desired.

The identification of subdominant, as opposed to dominant epitopes is desired. In the nomenclature adopted here (*see,* Sercarz, *et al.,* (1993), *supra*), a "dominant epitope" induces a response upon immunization with whole native antigens. Such a response is cross-reactive *in vitro* with the peptide epitope. A "cryptic epitope" elicits a response by peptide immunization, but is not cross-reactive *in vitro* when intact whole protein is used as an antigen. Finally, a "subdominant epitope" is an epitope which evokes little or no response upon immunization with whole antigens, but for which a response can be obtained by peptide immunization, and this response (unlike the case of cryptic epitopes) is detected when whole protein is used to recall the response *in vitro.*

The concept of dominance and subdominance is relevant to immunotherapy of viral disease and cancer. In the course of chronic viral disease, recruitment of subdominant epitopes can be crucial for successful clearance of the infection, especially if dominant CTL specificities have been inactivated by functional tolerance, suppression, mutation of viruses and other mechanisms (Franco, et al., Current Opinion in Immunology, 7:524-531, (1995)). Furthermore, in the case of cancer and tumor antigens, it appears that CTL recognizing at least some of the highest binding peptides might have been functionally inactivated by tolerance and suppression, and lower binding affinity peptides are preferentially recognized.

In particular, it has been noted that a significant number of epitopes derived from known non-viral tumor associated antigens (TAA) bind HLA Class I with intermediate affinity (IC50% in the 50-500 mM range). It has been found that 8 of 15 known TAA peptides recognized by tumor infiltrating lymphocytes (TIL) or CTL bound in the 50-500 mM range. These data are in contrast with estimates that 90% of known viral antigens that were recognized as peptides bound HLA with IC50% of 50 µM or less while only approximately 10% bound in the 50-500 mM range (Sette, et al., J. Immunol., 153:5586-5592 (1994)). This phenomenon is probably due in the cancer setting to elimination, or functional inhibition of the CTL recognizing several of the highest binding peptides, presumably because of T cell tolerization events.

Herein described are methods for modulating binding affinity of immunogenic peptides by selection of desired residues in the primary and secondary anchor positions. As explained in detail below, a supermotif for enhanced binding to A3-like alleles is provided here. Depending on the desired affect on binding affinity, the anchor residues in a desired peptide are substituted. Examples of modulations that may be achieved include increased affinity for a particular allele (*e.g.,* by substitution of secondary anchor residues specific for the allele), increased cross-reactivity among different alleles (*e.g*., by substitution of secondary anchor residues shared by more than one allele), and production of a subdominant epitope (*e.g*., by substitution of residues which increase affinity but are not present on the immunodominant epitope).

Epitopes on a number of potential target proteins can be used. Examples of suitable antigens include prostate specific antigen (PSA), hepatitis B core and surface antigens (HBVc, HBVs) hepatitis C antigens, Epstein-Barr virus antigens, melanoma antigens (e.g., MAGE-1), human immunodeficiency virus (HIV) antigens and human papilloma virus (HPV) antigens. Exemplary fungal antigens include those derived from *Candida albicans Cryptococcus neoformans, Coccidoides spp. , Histoplasma spp.,* and *Aspergillus fumigatis.* Parasitic antigens include those derived from *Plasmodium spp., trypanosoma spp., Schistosoma spp., leishmania spp* and the like.

The preparation and evaluation of peptides are described in PCT publications WO 94/20127 and WO 94/03205. Briefly, peptides comprising epitopes from a particular antigen are synthesized and tested for their ability to bind to the appropriate MHC molecules in assays using, for example, purified class I molecules and radioiodonated peptides and/or cells expressing empty class I molecules by, for instance, immunofluorescent staining and flow microfluorimetry, peptide-dependent class I assembly assays, and inhibition of CTL recognition by peptide competition. Those peptides that bind to the class I molecule are further evaluated for their ability to serve as targets for CTLs derived from infected or immunized individuals, as well as for their capacity to induce primary *in vitro* or *in vivo* CTL responses that can give rise to CTL populations capable of reacting with selected target cells associated with a disease.

Throughout this disclosure, results are expressed in terms of IC50's. Given the conditions in which the assays are run (i.e., limiting MHC and labeled peptide concentrations), these values approximate K_{D} values. It should be noted that IC50 values can change, often dramatically, if the assay conditions are varied, and depending on the particular reagents used (*e.g.,* MHC preparation, etc.). For example, excessive concentrations of MHC will increase the apparent measured IC50 of a given ligand. As used herein, "high affinity" is defined here as binding with an IC50 (or K_{D}) of less than 50nM. "Intermediate affinity" is binding with an IC50 (or K_{D}) of between about 50 and about 500nM. Assays for determining binding are described in detail in PCT publications WO 94/20127 and WO 94/03205.

An alternative way of expressing the binding data is as a relative value to a reference peptide. The reference peptide is included in every assay. As a particular assay becomes more, or less, sensitive, the IC50's of the peptides tested may change somewhat. However, the binding relative to the reference peptide will not change. For example, in an assay run under conditions such that the IC50 of the reference peptide increases 10-fold, all IC50 values will also shift approximately 10-fold. Therefore, to avoid ambiguities, the assessment of whether a peptide is a good, intermediate, weak, or negative binder should be based on it's IC50, relative to the IC50 of the standard peptide.

The nomenclature used to describe peptide compounds follows the conventional practice wherein the amino group is presented to the left (the N-terminus) and the carboxyl group to the right (the C-terminus) of each amino acid residue. In the formulae, the amino- and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G.

The immunogenic peptides can be prepared synthetically, or by recombinant DNA technology or from natural sources such as whole viruses or tumors. Although the peptide will preferably be substantially free of other naturally occurring host cell proteins and fragments thereof, in some embodiments the peptides can be synthetically conjugated to native fragments or particles.

The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Desirably, the peptide will be as small as possible while still maintaining substantially all of the biological activity of the large peptide. When possible, it may be desirable to optimize peptides to a length of about 8 to about 20 amino acid residues, typically 9 to 15, and preferably 9 to 10, commensurate in size with endogenously processed viral peptides or tumor cell peptides that are bound to MHC class I molecules on the cell surface. The preferred supermotifs disclosed here suitable for peptides of about 9 residues in length. The identification of supermotifs, primary anchors, and secondary anchors for peptides of other lengths (*e.g*., 8, 10, and 11 residues) can be carried out using the techniques described here.

Peptides having the desired activity may be modified as necessary to provide certain desired attributes, e.g., improved pharmacological characteristics, while increasing or at least retaining substantially all of the biological activity of the unmodified peptide to bind the desired MHC molecule and activate the appropriate T cell. For instance, the peptides may be subject to various changes, such as substitutions, either conservative or non-conservative, where such changes might provide for certain advantages in their use, such as improved MHC binding. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr. The effect of single amino acid substitutions may also be probed using D-amino acids. Such modifications may be made using well known peptide synthesis procedures, as described in e.g., Merrifield, Science 232:341-347 (1986), and Stewart and Young, Solid Phase Peptide Synthesis, (Rockford, Ill., Pierce), 2d Ed. (1984).

The peptides can also be modified by extending or decreasing the compound's amino acid sequence, e.g., by the addition or deletion of amino acids. The peptides or analogs can also be modified by altering the order or composition of certain residues, it being readily appreciated that certain amino acid residues essential for biological activity, e.g., those at critical contact sites or conserved residues, may generally not be altered without an adverse effect on biological activity. The non-critical amino acids need not be limited to those naturally occurring in proteins, such as L-α-amino acids, or their D-isomers, but may include non-natural amino acids as well, such as β-γ-δ-amino acids, as well as many derivatives of L-α-amino acids.

Typically, a series of peptides with single amino acid substitutions are employed to determine the effect of electrostatic charge, hydrophobicity, etc. on binding. For instance, a series of positively charged (e.g., Lys or Arg) or negatively charged (e.g., Glu) amino acid substitutions are made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (e.g., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding.

Amino acid substitutions are typically of single residues. Substitutions, deletions, insertions or any combination thereof may be combined to arrive at a final peptide. Substitutional variants are those in which at least one residue of a peptide has been removed and a different residue inserted in its place. Such substitutions generally are made in accordance with the following Table 1 when it is desired to finely modulate the characteristics of the peptide.

**TABLE 1**

| *Original Residue* | *Exemplary Substitution* |
|---|---|
| Ala | Ser |
| Arg | Lys, His |
| Asn | Gln |
| Asp | Glu |
| Cys | Ser |
| Gln | Asn |
| Glu | Asp |
| Gly | Pro |
| His | Lys; Arg |
| Ile | Leu; Val |
| Leu | Ile; Val |
| Lys | Arg; His |
| Met | Leu; Ile |
| Phe | Tyr; Trp |
| Ser | Thr |
| Thr | Ser |
| Trp | Tyr; Phe |
| Tyr | Trp; Phe |
| Val | Ile; Leu |

Substantial changes in function (e.g., affinity for MHC molecules or T cell receptors) are made by selecting substitutions that are less conservative than those in Table 1, i.e., selecting residues that differ more significantly in their effect on maintaining (a) the structure of the peptide backbone in the area of the substitution, for example as a sheet or helical conformation, (b) the charge or hydrophobicity of the molecule at the target site or (c) the bulk of the side chain. The substitutions which in general are expected to produce the greatest changes in peptide properties will be those in which (a) hydrophilic residue, e.g. seryl, is substituted for (or by) a hydrophobic residue, e.g. leucyl, isoleucyl, phenylalanyl, valyl or alanyl; (b) a residue having an electropositive side chain, e.g., lysl, arginyl, or histidyl, is substituted for (or by) an electronegative residue, e.g. glutamyl or aspartyl; or (c) a residue having a bulky side chain, e.g. phenylalanine, is substituted for (or by) one not having a side chain, e.g., glycine.

The peptides may also comprise isosteres of two or more residues in the immunogenic peptide. An isostere as defined here is a sequence of two or more residues that can be substituted for a second sequence because the steric conformation of the first sequence fits a binding site specific for the second sequence. The term specifically includes peptide backbone modifications well known to those skilled in the art. Such modifications include modifications of the amide nitrogen, the α-carbon, amide carbonyl, complete replacement of the amide bond, extensions, deletions or backbone crosslinks. *See, generally,* Spatola, Chemistry and Biochemistry of Amino Acids, Peptides and Proteins, Vol. VII (Weinstein ed., 1983).

Modifications of peptides with various amino acid mimetics or unnatural amino acids are particularly useful in increasing the stability of the peptide *in vivo.* Stability can be assayed in a number of ways. For instance, peptidases and various biological media, such as human plasma and serum, have been used to test stability. *See, e.g*., Verhoef et al., Eur. J. Drug Metab. Pharmacokin 11:291-302 (1986). Half life of the peptides is conveniently determined using a 25 % human serum (v/v) assay. The protocol is generally as follows. Pooled human serum (Type AB, non-heat inactivated) is delipidated by centrifugation before use. The serum is then diluted to 25% with RPMI tissue culture media and used to test peptide stability. At predetermined time intervals a small amount of reaction solution is removed and added to either 6% aqueous trichloracetic acid or ethanol. The cloudy reaction sample is cooled (4°C) for 15 minutes and then spun to pellet the precipitated serum proteins. The presence of the peptides is then determined by reversed-phase HPLC using stability-specific chromatography conditions.

The peptides or analogs thereof which have CTL stimulating activity may be modified to provide desired attributes other than improved serum half life. For instance, the ability of the peptides to induce CTL activity can be enhanced by linkage to a sequence which contains at least one epitope that is capable of inducing a T helper cell response. Particularly preferred immunogenic peptides/T helper conjugates are linked by a spacer molecule. The spacer is typically comprised of relatively small, neutral molecules, such as amino acids or amino acid mimetics, which are substantially uncharged under physiological conditions. The spacers are typically selected from, e.g., Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues. Alternatively, the CTL peptide may be linked to the T helper peptide without a spacer.

The immunogenic peptide may be linked to the T helper peptide either directly or via a spacer either at the amino or carboxy terminus of the CTL peptide. The amino terminus of either the immunogenic peptide or the T helper peptide may be acylated. The T helper peptides used can be modified in the same manner as CTL peptides. For instance, they may be modified to include D-amino acids or be conjugated to other molecules such as lipids, proteins, sugars and the like. Exemplary T helper peptides include tetanus toxoid 830-843, influenza 307-319, malaria circumsporozoite 382-398 and 378-389.

Alternatively, the T helper peptide is one that is recognized by T helper cells in the majority of the population. This can be accomplished by selecting amino acid sequences that bind to many, most, or all of the MHC class II molecules. These are known as "loosely MHC-restricted" or "promiscuous" T helper sequences. Examples of amino acid sequences that are promiscuous include sequences from antigens such as Tetanus toxin at positions 830-843 (QYIKANSKFIGITE), *Plasmodium falciparum* CS protein at positions 378-398 (DIEKKIAKMEKASSVFNVVNS), and Streptococcus 18kD protein at positions 1-16 (GAVDSILGGVATYGAA).

Alternatively, it is possible to prepare synthetic peptides capable of stimulating T helper lymphocytes, in a loosely MHC-restricted fashion, using amino acid sequences not found in nature (*see, e.g.,* PCT publication WO 95/07707). These synthetic compounds called Pan-DR-binding epitope (PADRE) are designed on the basis of their binding activity to most, HLA-DR (human MHC class II) molecules. For instance, a peptide having the formula: aKXVWANTLKAAa, where X = cyclohexylalanine, phenylalanine, or tyrosine, and a = D-alanine or L-alanine, has been found to bind to most HLA-DR alleles, and to stimulate the response of T helper lymphocytes from most individuals, regardless of their HLA type. T helper epitopes can also be modified to increase their biological effect. For example, peptides presenting T helper epitopes can contain D-amino acids to increase their resistance to proteases and thus extend their serum half-life. Also, the T helper peptides can be conjugated to other molecules such as lipids, proteins or sugars, or any other synthetic compound, to increase their biological activity. Specifically, the T helper peptide can be conjugated to one or more palmitic acid chains at either the amino or carboxyl termini.

In some embodiments it may be desirable to include in the pharmaceutical compositions at least one component which primes CTL. Lipids have been identified as agents capable of priming CTL *in vivo* against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, *E. coli* lipoproteins, such as tripalmitoyl-S-glycerylcysteinlyseryl- serine (P₃CSS) I can be used to prime virus specific CTL when covalently attached to an appropriate peptide. See, Deres et al., Nature 342:561-564 (1989). Peptides can be coupled to P₃CSS, for example, and the lipopeptide administered to an individual to specifically prime a CTL response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In addition, additional amino acids can be added to the termini of a peptide to provide for ease of linking peptides one to another, for coupling to a carrier support, or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. Modification at the C terminus in some cases may alter binding characteristics of the peptide. In addition, the peptide or oligopeptide sequences can differ from the natural sequence by being modified by terminal-NH₂ acylation, e.g., by alkanoyl (C₁-C₂₀) or thioglycolyl acetylation, terminal-carboxyl amidation, e.g., ammonia, methylamine, etc. In some instances these modifications may provide sites for linking to a support or other molecule.

The peptides can be prepared in a wide variety of ways. Because of their relatively short size, the peptides can be synthesized in solution or on a solid support in accordance with conventional techniques. Various automatic synthesizers are commercially available and can be used in accordance with known protocols. See, for example, Stewart and Young, Solid Phase Peptide Synthesis, 2d. ed., Pierce Chemical Co. (1984), *supra.*

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook et al., Molecular Cloning, a Laboratory Manual, Cold Spring Harbor Press, Cold Spring Harbor, New York (1982). Thus, fusion proteins which comprise one or more peptide sequences disclosed can be used to present the appropriate T cell epitope.

As the coding sequence for peptides of the length contemplated herein can be synthesized by chemical techniques, for example, the phosphotriester method of Matteucci et al., J. Am. Chem. Soc. 103:3185 (1981), modification can be made simply by substituting the appropriate base(s) for those encoding the native peptide sequence. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast or mammalian cell hosts may also be used, employing suitable vectors and control sequences.

The peptides and pharmaceutical and vaccine compositionsreferred to herein are useful for administration to mammals, particularly humans, to treat and/or prevent viral infection and cancer. Examples of diseases which can be treated using the immunogenic peptides include prostate cancer, hepatitis B, hepatitis C, AIDS, renal carcinoma, cervical carcinoma, lymphoma, CMV and condlyloma acuminatum. In addition, the peptides can be used to treat any number of infections diseases, such as viral, fungal, and parasitic infections. Suitable antigens are disclosed, for instance, in WO 94/20127 and WO 94/03205.

As noted above, the peptides induce CTL immune responses when contacted with a CTL specific to an epitope on the peptide. The manner in which the peptide is contacted with the CTL is not critical, however. For instance, the peptide can be contacted with the CTL either *in vivo* or *in vitro.* If the contacting occurs *in vivo,* the peptide itself can be administered to the patient or other vehicles (*e.g*., DNA vectors encoding one or more peptide, viral vectors encocoding the peptides, liposomes and the like) can be used, as described below.

For pharmaceutical compositions, the immunogenic peptides, or DNA encoding them, are administered to an individual already suffering from cancer or infected with the pathogen of interest. The peptides or DNA encoding them can be administered individually or as fusions of one or more of the peptide sequences disclosed here. Those in the incubation phase or the acute phase of infection can be treated with the immunogenic peptides separately or in conjunction with other treatments, as appropriate. In therapeutic applications, compositions are administered to a patient in an amount sufficient to elicit an effective CTL response to the virus or tumor antigen and to cure or at least partially arrest symptoms and/or complications. An amount adequate to accomplish this is defined as "therapeutically effective dose." Amounts effective for this use will depend on, e.g., the particular composition administered, the manner of administration, the stage and severity of the disease being treated, the weight and general state of health of the patient, and the judgment of the prescribing physician, but generally range for the initial immunization (that is for therapeutic or prophylactic administration) from about 1.0 µg to about 5000 µg of peptide for a 70 kg patient, followed by boosting dosages of from about 1.0 µg to about 1000 µg of peptide pursuant to a boosting regimen over weeks to months depending upon the patient's response and condition by measuring specific CTL activity in the patient's blood. It must be kept in mind that the peptides and compositions disclosed may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

For therapeutic use, administration should begin at the first sign of viral infection or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions herein described may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide or other compositions can also be used for the treatment of chronic infection and to stimulate the immune system to eliminate pathogen-infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 µg to about 5000 µg, preferably about 5 µg to 1000 µg for a 70 kg patient per dose. Immunizing doses followed by boosting doses at established intervals, e.g., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus disclosed herein are compositions for parenteral administration which comprise a solution of the immunogenic peptides dissolved or suspended in an acceptable carrier, preferably an aqueous carrier. A variety of aqueous carriers may be used, e.g., water, buffered water, 0.8% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of CTL stimulatory peptides in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The peptides may also be administered via liposomes, which serve to target the peptides to a particular tissue, such as lymphoid tissue, or targeted selectively to infected cells, as well as increase the half-life of the peptide composition. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes either filled or decorated with a desired peptide can be directed to the site of lymphoid cells, where the liposomes then deliver the selected therapeutic/immunogenic peptide compositions. Liposomes are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., An. Rev. Biophys. Bioeng. 9:467 (1980), U.S. Patent Nos. 4,235,871, 4,501,728, 4,837,028, and 5,019,369.

For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, *inter alia,* the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides, and more preferably at a concentration of 25%-75%.

For aerosol administration, the immunogenic peptides are preferably supplied in finely divided form along with a surfactant and propellant. Typical percentages of peptides are 0.01 %-20% by weight, preferably 1 %-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

There is also reference herein to vaccines which contain as an active ingredient an immunogenically effective amount of one or more immunogenic peptide as described herein. The peptide(s) may be introduced into a host, including humans, linked to its own carrier or as a homopolymer or heteropolymer of active peptide units. Such a polymer has the advantage of increased immunological reaction and, where different peptides are used to make up the polymer, the additional ability to induce antibodies and/or CTLs that react with different antigenic determinants of the virus or tumor cells. Useful carriers are well known in the art, and include, e.g., thyroglobulin, albumins such as human serum albumin, tetanus toxoid, polyamino acids such as poly(lysine:glutamic acid); influenza, hepatitis B virus core protein, hepatitis B virus recombinant vaccine and the like. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, CTL responses can be primed by conjugating peptides to lipids, such as P₃CSS. Upon immunization with a peptide composition as described herein, via injection, aerosol, oral, transdermal or other route, the immune system of the host responds to the vaccine by producing large amounts of CTLs specific for the desired antigen, and the host becomes at least partially immune to later infection, or resistant to developing chronic infection.

Vaccine compositions containing the peptides are administered to a patient susceptible to or otherwise at risk of viral infection or cancer to elicit an immune response against the antigen and thus enhance the patient's own immune response capabilities. Such an amount is defined to be an "immunogenically effective dose." In this use, the precise amounts again depend on the patient's state of health and weight, the mode of administration, the nature of the formulation, etc., but generally range from about 1.0 µg to about 5000 µg per 70 kilogram patient, more commonly from about 10 µg to about 500 µg mg per 70 kg of body weight.

In some instances it may be desirable to combine the peptide vaccines described herein, with vaccines which induce neutralizing antibody responses to the virus of interest, particularly to viral envelope antigens.

For therapeutic or immunization purposes, the peptides can also be expressed by attenuated viral hosts, such as vaccinia or fowlpox. This approach involves the use of vaccinia virus as a vector to express nucleotide sequences that encode the peptides. Upon introduction into an acutely or chronically infected host or into a non-infected host, the recombinant vaccinia virus expresses the immunogenic peptide, and thereby elicits a host CTL response. Vaccinia vectors and methods useful in immunization protocols are described in, e.g., U.S. Patent No. 4,722,848. Another vector is BCG (Bacille Calmette Guerin). BCG vectors are described in Stover et al. (Nature 351:456-460 (1991)). A wide variety of other vectors useful for therapeutic administration or immunization of the peptides of the invention, e.g., *Salmonella Typhi* vectors and the like, will be apparent to those skilled in the art from the description herein.

Antigenic peptides may be used to elicit CTL *ex vivo,* as well. The resulting CTL, can be used to treat chronic infections (viral or bacterial) or tumors in patients that do not respond to other conventional forms of therapy, or will not respond to a peptide vaccine approach of therapy. *Ex vivo* CTL responses to a particular pathogen (infectious agent or tumor antigen) are induced by incubating in tissue culture the patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell).

The DNA encoding one or more of the peptides can also be admisitered to the patient. This approach is described, for instance, in Wolff et. al., Science 247: 1465-1468 (1990) as well as U.S. Patent Nos. 5,580,859 and 5,589,466.

A preferred means of administering nucleic acids encoding the peptides uses minigene constructs encoding multiple epitopes. To create a DNA sequence encoding the selected CTL epitopes (minigene) for expression in human cells, the amino acid sequences of the epitopes are reverse translated. A human codon usage table is used to guide the codon choice for each amino acid. These epitope-encoding DNA sequences are directly adjoined, creating a continuous polypeptide sequence. To optimize expression and/or immunogenicity, additional elements can be incorporated into the minigene design. Examples of amino acid sequence that could be reverse translated and included in the minigene sequence include: helper T lymphocyte epitopes, a leader (signal) sequence, and an endoplasmic reticulum retention signal. In addition, MHC presentation of CTL epitopes may be improved by including synthetic (e.g. poly-alanine) or naturally-occurring flanking sequences adjacent to the CTL epitopes.

The minigene sequence is converted to DNA by assembling oligonucleotides that encode the plus and minus strands of the minigene. Overlapping oligonucleotides (30-100 bases long) are synthesized, phosphorylated, purified and annealed under appropriate conditions using well known techniques. he ends of the oligonucleotides are joined using T4 DNA ligase. This synthetic minigene, encoding the CTL epitope polypeptide, can then cloned into a desired expression vector.

Standard regulatory sequences well known to those of skill in the art are included in the vector to ensure expression in the target cells. Several vector elements are required: a promoter with a down-stream cloning site for minigene insertion; a polyadenylation signal for efficient transcription termination; an *E. coli* origin of replication; and an *E. coli* selectable marker (e.g. ampicillin or kanamycin resistance). Numerous promoters can be used for this purpose, *e.g*., the human cytomegalovirus (hCMV) promoter. *See,* U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences can also be considered for increasing minigene expression. It has recently been proposed that immunostimulatory sequences (ISSs or CpGs) play a role in the immunogenicity of DNA vaccines. These sequences could be included in the vector, outside the minigene coding sequence, if found to enhance immunogenicity.

In some embodiments, a bicistronic expression vector, to allow production of the minigene-encoded epitopes and a second protein included to enhance or decrease immunogenicity can be used. Examples of proteins or polypeptides that could beneficially enhance the immune response if co-expressed include cytokines (e.g., IL2, IL12, GM-CSF), cytokine-inducing molecules (e.g. LeIF) or costimulatory molecules. Helper (HTL) epitopes could be joined to intracellular targeting signals and expressed separately from the CTL epitopes. This would allow direction of the HTL epitopes to a cell compartment different than the CTL epitopes. If required, this could facilitate more efficient entry of HTL epitopes into the MHC class II pathway, thereby improving CTL induction. In contrast to CTL induction, specifically decreasing the immune response by co-expression of immunosuppressive molecules (e.g. TGF-β) may be beneficial in certain diseases.

Once an expression vector is selected, the minigene is cloned into the polylinker region downstream of the promoter. This plasmid is transformed into an appropriate *E. coli* strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

Therapeutic quantities of plasmid DNA are produced by fermentation in *E. coli,* followed by purification. Aliquots from the working cell bank are used to inoculate fermentation medium (such as Terrific Broth), and grown to saturation in shaker flasks or a bioreactor according to well known techniques. Plasmid DNA can be purified using standard bioseparation technologies such as solid phase anion-exchange resins supplied by Quiagen. If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). This approach, known as "naked DNA," is currently being used for intramuscular (IM) administration in clinical trials. To maximize the immunotherapeutic effects of minigene DNA vaccines, an alternative method for formulating purified plasmid DNA may be desirable. A variety of methods have been described, and new techniques may become available. Cationic lipids can also be used in the formulation (*see, e.g.,* as described by Debs and Zhu (1993) WO 93/24640; Mannino and Gould-Fogerite (1988) BioTechniques 6(7): 682-691; Rose U.S. Pat No. 5,279,833; Brigham (1991) WO 91/06309; and Felgner et al. (1987) Proc. Natl. Acad. Sci. USA 84: 7413-7414). In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

The nucleic acids can also be administered using ballistic delivery as described, for instance, in U.S. Patent No. 5,204,253. Particles comprised solely of DNA can be administered. Alternatively, DNA can be adhered to particles, such as gold particles.

Target cell sensitization can be used as a functional assay for expression and MHC class I presentation of minigene-encoded CTL epitopes. The plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 labeled and used as target cells for epitope-specific CTL lines. Cytolysis, detected by 5 1 Cr release, indicates production of MHC presentation of minigene-encoded CTL epitopes.

*In vivo* immunogenicity is a second approach for functional testing of minigene DNA formulations. Transgenic mice expressing appropriate human MHC molecules are immunized with the DNA product. The dose and route of administration are formulation dependent (e.g. IM for DNA in PBS, IP for lipid-complexed DNA). Twenty-one days after immunization, splenocytes are harvested and restimulated for I week in the presence of peptides encoding each epitope being tested. These effector cells (CTLs) are assayed for cytolysis of peptide-loaded, chromium-51 labeled target cells using standard techniques. Lysis of target cells sensitized by MHC loading of peptides corresponding to minigene-encoded epitopes demonstrates DNA vaccine function for *in vivo* induction of CTLs.

The peptides may also find use as diagnostic reagents. For example, a peptide may be used to determine the susceptibility of a particular individual to a treatment regimen which employs the peptide or related peptides, and thus may be helpful in modifying an existing treatment protocol or in determining a prognosis for an affected individual. In addition, the peptides may also be used to predict which individuals will be at substantial risk for developing chronic infection.

### Example 1

### A3-like supertype binding

This example provides refined motifs for each of the A3-like alleles A3, All, A*3101, A*3301, and A*6801, outlining secondary anchor-binding specificities. The motifs were derived by calculating at each non-anchor position along the peptide sequence the average relative binding capacity of peptides carrying each of the 20 common amino acids, grouped according to individual chemical similarities.

### Materials and Methods

***Class I purification**.* The following Epstein-Barr virus (EBV)-transformed homozygous cell lines were used as sources of class 1 molecules: GM3107 (A3, B7; Human Genetic Mutant Repository); BVR (A11, B35.3, Cw4; Human Genetic Mutant Repository); SPACH (A31, B62, Cw1/3; ASHI Repository Collection); and LWAGS (A*3301, B14, Cw8; ASHI Repository Collection) (Bodmer, et al., Hum Immunol 43:149 (1995)). A C1R transfectant characterized by Dr. Walter Storkus (University of Pittsburgh) was used for the isolation of A*6801. Cell lines were maintained as previously described (Sidney, et al., J Immunol 154:247 (1995); Sette, et al., Mol Immunol 31:813 (1994)).

Cell lysates were prepared and class I molecules purified as previously described (Sidney, et al., J Immunol 154:247 (1995); Sette, et al., Mol Immunol 31:813 (1994)). Briefly, cells were lysed at a concentration of 10⁸ cells/ml in 50 mM Tris-HCL, pH 8.5, containing 1% NP-40 (Fluka Biochemika, Buchs, Switzerland), 150 mM NaCl, 5 mM EDTA, and 2 mM PMSF. The lysates were passed through 0.45 µM filters and cleared of nuclei and debris by centrifugation at 10,000 g for 20 minutes. Major histocompatibility complex (MHC) molecules were then purified by affinity chromatography. Columns of inactivated Sepharose CL4B and Protein A Sepharose were used as precolumns. The cell lysate was depleted of HLA-B and HLA-C molecules by repeated passage over Protein A Sepharose beads conjugated with the anti-HLA(B,C) antibody B1.23.2 (Rebai, et al., Tissue Antigens 22:107 (1983)). Typically two to four passages were required for effective depletion. Subsequently, the anti HLA(A,B,C) antibody W6/32 (Barnstable, et al., Cell 14:9 (1978)) was used to capture HLA-A molecules. Protein purity, concentration, and effectiveness of depletion steps were monitored by sodium dodecyl sulfate-polyacrylamide gel electrophoresis (SDS-PAGE).

***Binding assays and supermotif definition.*** Quantitative assays for the binding of peptides to soluble class I molecules on the basis of the inhibition of binding of a radiolabeled standard probe peptide to detergent solubilized MHC molecules were performed as previously described (Kubo, et al., J Immunol 152:3913 (1994); Kast, et al., J Immunol 152:3904 (1994); Sidney, et al., J Immunol 154:247 (1995); Sette, et al., Mol Immunol 31:813 (1994); Ruppert, et al., Cell 74:929 (1993)). Briefly, 1-10 nM of radiolabeled probe peptide, iodinated by the Chloramine T method (Greenwood, et al., Biochem J 89:114 (1963)), was co-incubated at room temperature with various amounts of MHC in the presence of 1 µM human β-microglobulin (Scripps Laboratories, San Diego, CA, USA) and a cocktail of protease inhibitors. At the end of a 2-day incubation period, the percent of MHC-bound radioactivity was determined by size exclusion gel filtration chromatography on a TSK 2000 column.

The A3CON1 peptide (sequence KVFPYALINK) (Kubo, et al., J Immunol 152:3913 (1994)) was used as the radiolabeled probe for the A3, A11, A31, and A*6801 assays. A T₇ → Y analogue of HBVc 141-151 (sequence STLPETYVVRR) (Missale, et al., J Exp Med 177:751 (1993)) was used as the radiolabeled probe for the A*3301 assay. In the case of competitive assays, the concentration of peptide yielding 50% inhibition of the binding of the radiolabeled probe peptide (IC50) was calculated. Peptides were usually tested at one or two high doses, and the IC50 of peptides yielding positive inhibition were determined in subsequent experiments, in which two to six further dilutions were tested, as necessary. MHC concentrations yielding approximately 15% binding of the radiolabled probe peptide were used for all competitive inhibition assays. Because under these conditions (label) < (MHC) and IC50 > (MHC), the measured IC50s are reasonable approximations of the true k_{D} values. Each competitor peptide was tested in two to four completely independent experiments. As a positive control, in each experiment the unlabeled version of the relevant radiolabeled probe was tested and its IC50 measured. The average IC50s of A3CON1 for the A3, A11, A31, and A*6801 assays were 11, 6, 18, and 8 nM, respectively. The average IC50 of eh HBVc 141-151 peptide in the A*3301 assay was 29 nM.

***Definition of HLA-A-specific secondary anchor motifs.*** A modification of the procedure used by Ruppert et al (Ruppert, et al., Cell 74:929 (1993)) to define A*0201 secondary anchor motifs was utilized. Briefly, allele-specific secondary anchor motifs were defined by assessing the effect on HLA binding of the 20 commonly occurring amino acids at each nonanchor position of 9-mer sequences. Assessment was made by calculating the average relative binding values for each position-amino acid combination (e.g., position 1, alanine; position 2, alanine, etc.). To overcome problems with the low occurrence of certain amino acids, some residues were grouped with others of similar nature as previously described (Ruppert, et al., Cell 74:929 (1993)). Residue types associated at a particular position with average binding capacities fourfold higher (or lower) than the overall average binding capacity of the whole 200-peptide set were considered associated with good (or poor) binding capacity.

***Peptide synthesis.*** Peptides were synthesized either synthesized as previously described (Ruppert, et al., Cell 74:929 (1993)), or purchased as crude material from Chiron Mimotopes (Chiron Corp., Australia). Synthesized peptides were purified to > 95% homogeneity by reverse-phase high-pressure liquid chromatography (HPLC). The purity of these synthetic peptides was assayed on an analytical reverse-phase column and their composition ascertained by amino acid analysis, sequencing, and/or mass spectrometry analysis.

***Calculation of phenotypic frequencies of HLA supertypes in various ethnic backgrounds and projected population coverage.*** Gene frequencies for each HLA allele were calculated from antigen or allele frequencies (Imanishi, et al., Proceedings of the Eleventh International Histocompatibility Workshop and Conference, Vol. 1, Tokyo, Oxford University Press (1992); Fernandez-Viña, et al., Hum Immunol 33:163 (1992)) utilizing the binomial distribution formulae gf = I - (SQRT(1 - af)) (Tiwari, et al., The HLA complex, In: HLA and Disease Associates, NY, Springer-Verlag (1985)). To obtain overall phenotypic frequencies, cumulative gene frequencies were calculated and the cumulative antigen frequencies derived by the use of the inverse formula (af = 1 - (1 - Cgf)²). As discussed below, where frequency data was not available at the level of DNA typing, correspondence to the serologically defined antigen frequencies was assumed. To obtain total potential population coverage no linkage disequilibrium was assumed and only alleles confirmed as belonging to each of the supertypes were included (minimal estimates). Estimates of total potential coverage achieved by interloci combinations were made by adding to the A coverage the proportion of the non-A covered population that could be expected to be covered by the B alleles considered (e.g., total = A + B*(1 - A)). Confirmed members of the A3-line supertype are A3, A11, A31, A*3301, and A*6801. Although the A3-like supertype may potentially include A32, A66, and A*7401, these alleles were not included in overall frequency calculations. Likewise, confirmed members of the A2-like supertype family A*0201, A*0202, A*0203, A*0204, A*0205, A*0206, A*6802, and A*6901 (potentially also A*3001). Finally, the B7-like supertype confirmed alleles are B7, B*3501-03, B51, B*5301, B*5401, B*5501-2, B*5601, B*6701, and B*7801 (potentially also B*1401, B*3504-06, B*4201, and B*5602).

### Results

***Structural analysis of the peptide-binding pockets of various HLA alleles.*** As mentioned above, previous studies have indicated that the HLA molecules A3, A11, and A*6801 are associated with specificity for ligands carrying small or hydrophobic residues in position 2, and positively charged C-termini (Kubo, et al., J Immunol 152:3913 (1994); Guo, et al., Nature 360:364 (1994); Falk, et al., Immunogenetics 40:238 (1994); Dibrino, et al., J Immunol 151:5930 (1993); DiBrino, et al., Proc Natl Acad Sci USA 90:1508 (1993); Zhang, et al., Proc Natl Acad Sci USA 90:2217 (1993); Sette, et al., Mol Immunol 31:813 (1994)). We decided to evaluate the possible structural basis for these apparent similarities in ligand specificity in more detail. To this end, because the side chains of the residue in position 2 and at the C-termini of antigenic peptides are known to contact the residues forming the B and F pockets of HLA class I molecules (Madden, et al., Cell 75:693 (1993); Saper, et al., J Mol Biol 219:277 (1991)), the residues that form these polymorphic pockets were tabulated for various HLA class I molecules. It was found that the HLA types which are known to recognize small or hydrophobic residues in position 2 (e.g., A*0101, A*0201, A*0301, A*1101, A*6801, and A*6802) and HLA types which recognize positively charged residues at the C-terminus (e.g., A*0301, A*1101, A*6801, and B*2705) of their peptide ligands shared certain key structural features. In particular, HLA molecules that bind small and hydrophobic residues in position 2 carried aliphatic residues (M or V) at positions 45 and 67, and potential hydrogen-bond-forming residues such as N and K, or H and Q at positions 66 and 70, respectively. All of these molecules also carried a Y residue at position 99. In contrast, class I molecules that exhibited different binding specificities differed in one or more of these positions. Similarly, only class I molecules that prefer positively charged C-termini carried D, T, L, and D at positions 77, 80, 81, and 116, respectively. In conclusion, this analysis suggested that a set of HLA class I molecules (A3, A11, and A*6801) share certain key structural features in their B and F pockets, and a common peptide ligand motif characterized by small or hydrophobic residues in position 2 and positively charged residues at the C-termini. At this point we tentatively designated these HLA class I molecules as part of an A3-like supertype, and the corresponding motif as the A3-like supermotif.

Analysis of other class I molecules for which motifs were unknown, revealed that A*3101, A*3301, A*3401, A*6601, and A*7401 also shared these same consensus sequences in their B and F pockets. These molecules were thus predicted to be part of the A3-like supertype. Recent data (Falk, et al., Immunogenetics 40:238 (1994)) have independently demonstrated that A31 and A33 are indeed characterized by an A3-like peptide motif.

***A3-like molecules exhibit overlapping primary anchor specificities and peptide-binding repertoires.*** To compare the range of motifs recognized by some of the most frequent A3-like molecules (A3, A11, A31, A*3301, and A*6801), more detailed molecular analysis of the main anchor (position 2 and C-terminal) binding specificities of these molecules was undertaken. A3- and A11-specific peptide-binding assays measuring the capacity of unlabeled synthetic peptides to inhibit the binding of a radiolabeled probe peptide to affinity-purified HLA class I molecules have been previously described (Kubo, et al., J Immunol 152:3913 (1994); Kast, et al., J Immunol 152:3904 (1994); Sette, et al., Mol Immunol 31:813 (1994)). Binding assays specific for A31, A*3301, and A*6801 were developed using similar approaches (Kubo, et al., J Immunol 152:3913 (1994); Kast, et al., J Immunol 152:3904 (1994); del Guercio, et al., J Immunol 154:685 (1995); Sidney, et al., J Immunol 154:247 (1995); Sette, et al., Mol Immunol 31:813 (1994); Ruppert, et al., Cell 74:929 (1993)).

Primary anchor specificities of the A3-like molecules were subsequently explored by testing a panel of peptides carrying substitutions at position 2 or 9 of a prototype poly-A peptide AXAAAAAAX for their inhibitory capacity for A3, A11, A31, A*3301, and A*6801. Each molecule expressed individual preferences, but in the majority of instances considered, significant binding was obtained when position 2 was occupied by either A, I, L, M, S, T, or V, and the C-terminus was either R or K. These data are in good agreement with pool sequencing data generated by us (Kubo, et al., J Immunol 152:3913 (1994)) and others (Falk, et al., Immunogenetics 40:238 (1994); Dibrino, et al., J Immunol 151:5930 (1993); DiBrino, et al., Proc Natl Acad Sci USA 90:1508 (1993)), and also extends in the cases of A31, A*3301, and A*6801 the definition of the primary anchor motifs. In conclusion, these data suggest that the A3-like primary anchor supermotif can be defined as A, I, L, M, S, T, or V in position 2, and either R or K at the C-terminus.

***A3-like molecules share overlapping peptide-binding repertoires.*** The extent to which the A3-like supertype primary anchor motif allows degenerate binding amongst the A3-like supertype molecules was examined next. A set of 200 naturally occurring 9-mer peptide sequences carrying residues A, I, L, M, S, T, or V in position 2 and K or R in position 9 was assembled. Other than the constraint that each possible anchor combination be represented in proportion to the natural frequency of the individual amino acids, the peptides comprising the set were randomly selected from viral and tumor antigen sequences. When each peptide was tested for its capacity to bind purified A3, A11, A31, A*3301, and A*6801 molecules, it was apparent that a unique binding pattern was associated with each allelic type (data not shown). For example, some peptides were rather selective, binding only one class I type, whereas certain other peptides cross-reacted rather extensively, binding four or five of the molecules tested.

It was found that, in general, about 10% (5% - 16%) of the peptide-HLA combinations were associated with good binding (IC50 ≤ 50 nM), and about 17% (11%-24%) with intermediate binding (IC50 50-500 nM) to any given allele. These frequencies of high and intermediate binding are similar to those previously noted for A*0201 pool-sequencing motif-containing peptides (Ruppert, et al., Cell 74:929 (1993)). Most notable, however, was the relatively high degree of cross-reactivity observed. Of the 127 peptides that were capable of binding to at least one A3-like molecule, 43 of them (34%) bound three or more of the A3-like supertype molecules. Four peptides bound all five of the A3-like molecules tested. By the comparison, in a set of 39 peptides which were tested for binding to five unrelated class I molecules (A*0101, A3, A24, and B7), only three (8%) bound to two molecules, and none bound to three or more molecules. The peptides identified as high or intermediate binders for at least four of the five A3-like molecules tested are listed in Table 2. In the table, good or intermediate binding capacities are defined as IC50 ≤ 500 mM, and are highlighted by *shading.* Taken together, these data demonstrate significant overlap in the binding repertoires of the A3-like supertype molecules, and validate the A3-like supertype primary anchor motif.

*The A3-like supermotif defines secondary anchor residues conferring degeneracy to potential peptide ligands.* As stated above, although the overlap in the binding repertoires of A3-like supertype molecules is significant, it is also far from complete; each A3-like molecule also retains substantial degrees of specificity. To understand the basis of the observed cross-reactivities, we attempted to define an A3-like supermotif that would describe those features of peptide ligands which are associated with A3-like supertype degeneracy, and which could be useful in predicting A3-like degenerate binders.

First, refined motifs for each of the A3-like alleles analyzed herein (A3, A11, A31, A*3301, and A*6801), outlining secondary anchor-binding specificities, were derived as described in the Materials and Methods. This approach is similar to the one previously used to define a refined A*0201 motif (Ruppert, et al., Cell 74:929 (1993)). The motifs were derived by calculating at each nonanchor position along the peptide sequence the average relative binding capacity of peptides carrying each of the 20 common amino acids, grouped according to individual chemical similarities. Representative of the data generated by this procedure, the values calculated for A3 are shown in Table 3A. Following this as an example, 21 different peptides were tested which possessed an aromatic residue (F, W, Y) in position 3 of their sequence. These peptides had an average relative binding capacity to A3 31.7-fold higher than the overall average of the 200-peptide set. By analogy to what was previously described in the case of A*0201, preferred and deleterious residues were defined as residues associated with average binding capacities that were fourfold greater than or fourfold less than, respectively, the overall average. Accordingly, aromatic residues in position 3 were considered "preferred" residues for A3 binding.

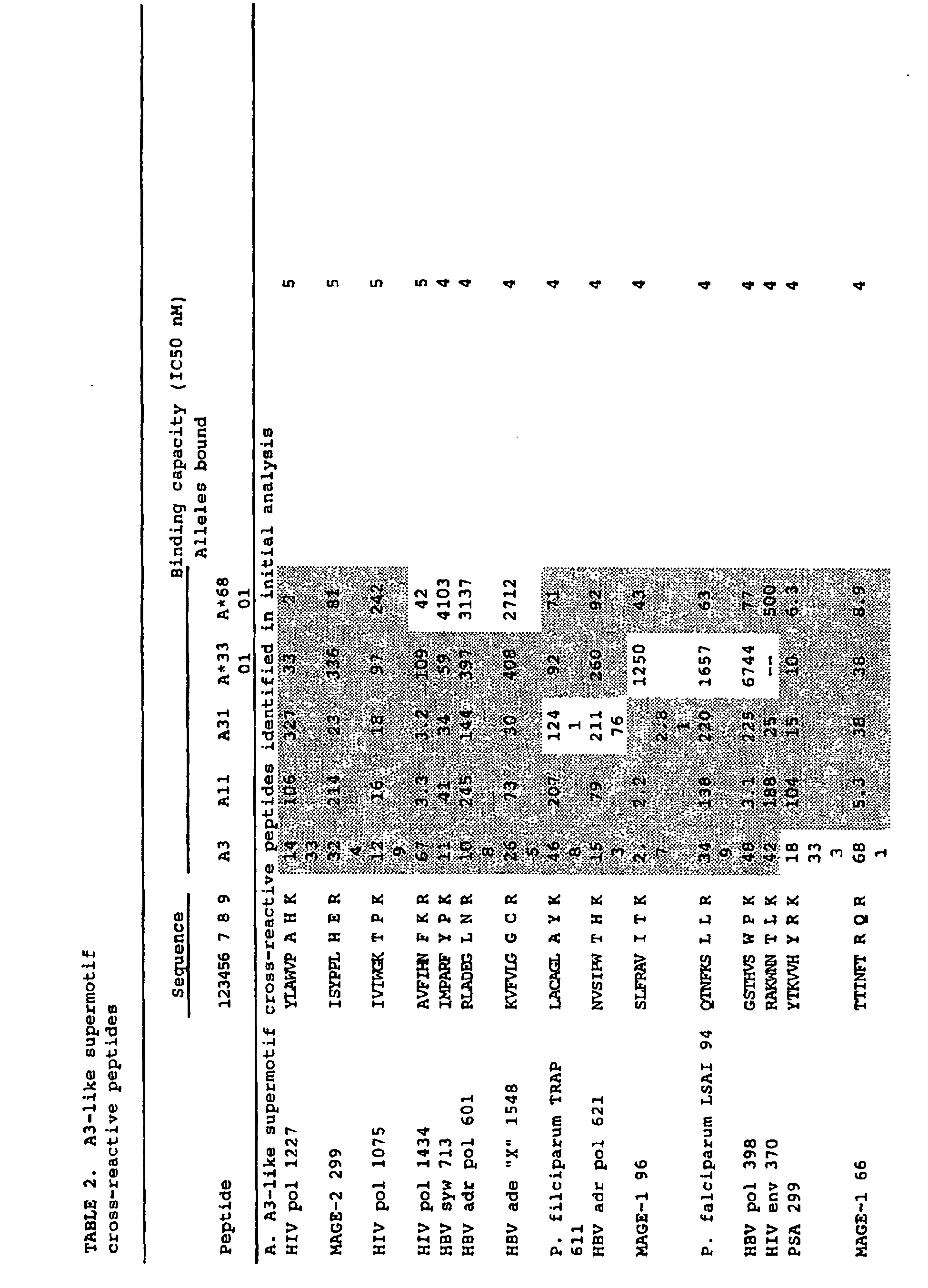

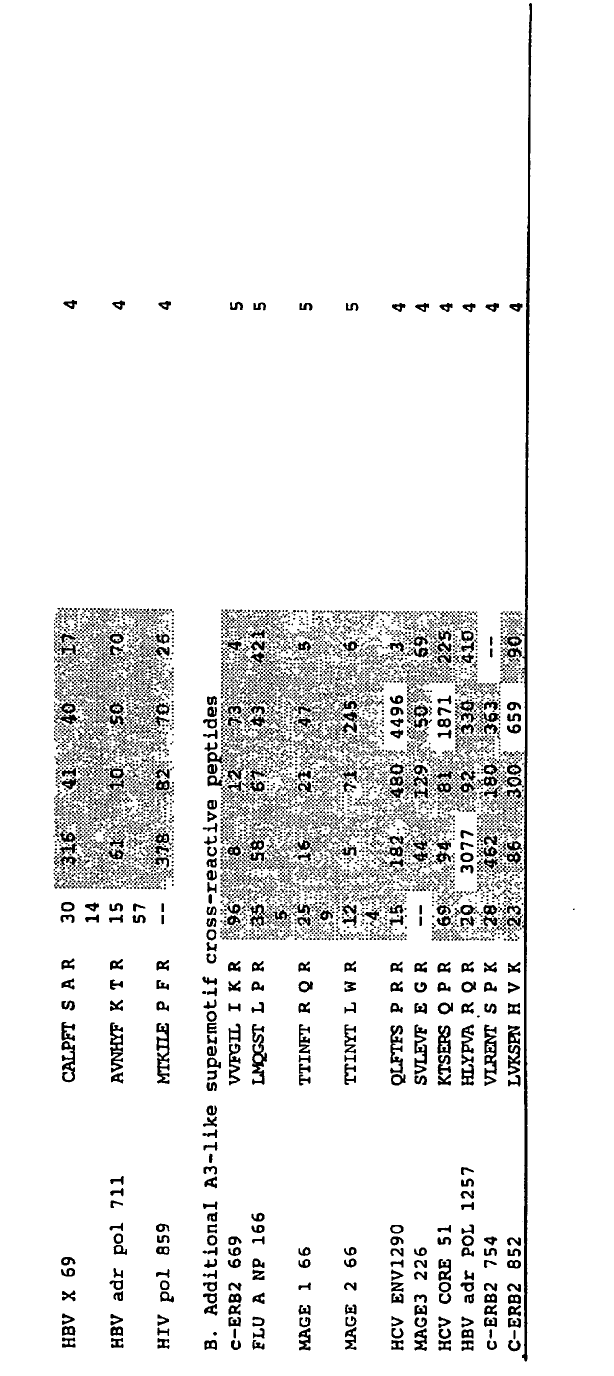

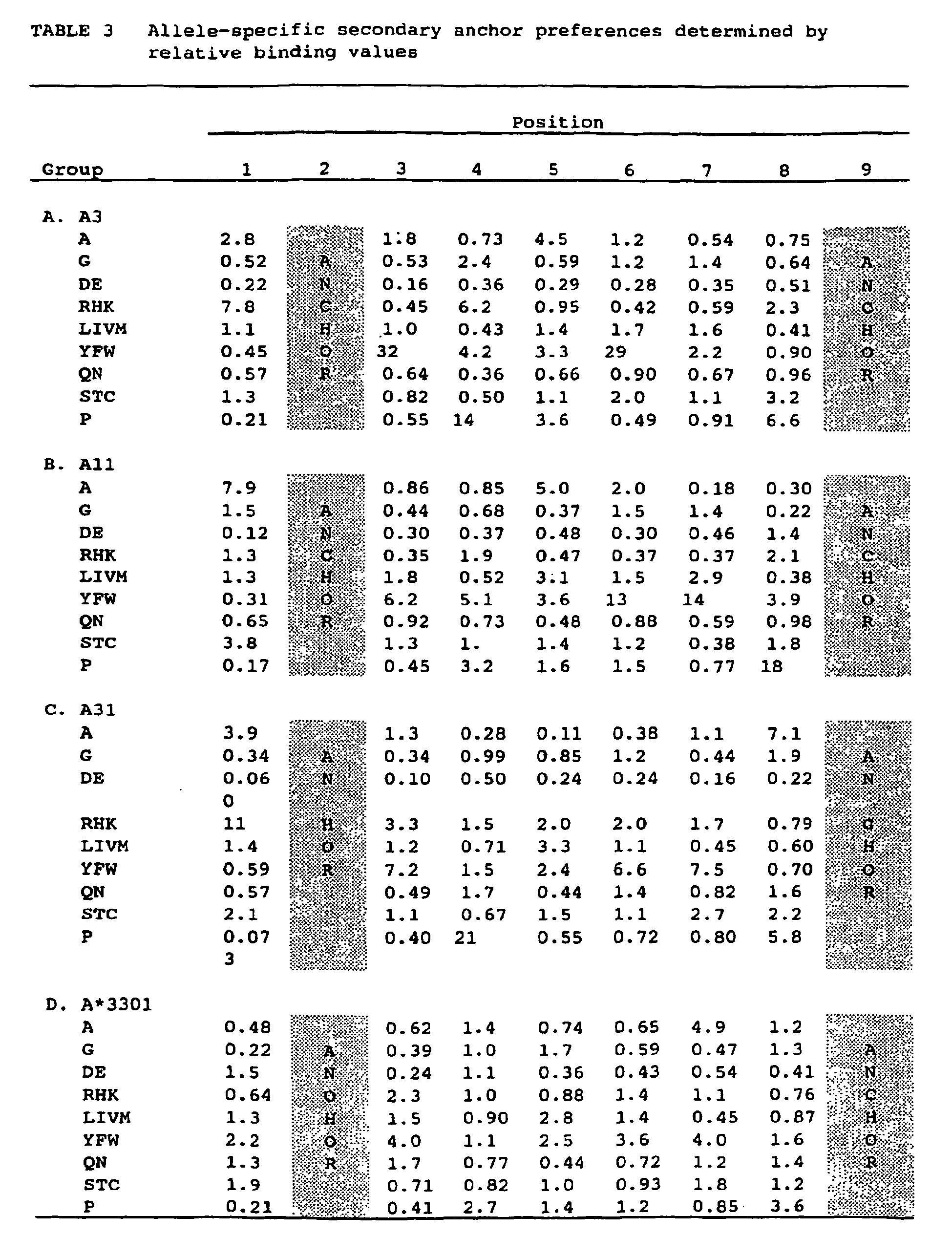

A similar analysis was performed for each allele (A3, A11, A31, A*3301) and was used to derive maps of allele-specific secondary anchor requirements for each position (Table 3B-E). Summaries of the modified motifs obtained for each allele of the A3-like supertype examined are shown in Fig. 1. Each molecule exhibited its own unique secondary anchor requirements. For example, positively charged residues(R, H, K) at position 4 were preferred by A3, but not by any other A3-like molecule. Similarly at position 8, glycine (G) was associated with poor binding capacity only for A11, whereas negative charges (D, E) were deleterious only for A31. Besides these types of unique allele-specific features, certain residues were associated with either poor or good binding in a majority of the molecules of the A3 supertype. For example, proline (P) in position 1 was deleterious for all five of the A3-like molecules tested. Aromatic residues (F, W, Y) in position 7 and proline in position 8 were preferred by four of the five molecules tested (Fig. 1).

On the basis of the various individual refined motifs, an A3-like supermotif was constructed. Residues deleterious for at least three of the five alleles considered were defined as deleterious residues in the supermotif. Conversely, residues preferred by at least three of the five alleles considered, but also not deleterious for any allele, were defined as preferred residues. The A3-like supermotif derived following this approach is shown in Fig. 2.

*A test of the efficacy of the A3-supermotif in predicting highly cross-reactive peptides.* To test the validity of the A3-like supermotif defined above, an additional set of 108 peptides not previously included in the analysis of supermotifs was tested for binding to A3, A11, A31, A*3301, and A*6801. This set included 30 peptides which had at least one preferred supermotif residue and no deleterious residues (supermotif positive), 43 peptides with at least one deleterious residue (supermotif negative), and 35 peptides with neither preferred nor deleterious residues (supermotif neutral). Of the 30 supermotif positive peptides, 27 (90%) bound to two or more A3-like molecules and 16 (53 %) bound to three or more molecules. By contrast, 18 (51 %) of 35 supermotif neutral peptides bound two or more A3 types, and eight (23 %) bound three or more molecules. Finally, the supermotif-negative peptides were much less capable of binding multiple alleles, with six (14 %) peptides binding two A3-like molecules, and no peptides binding three or more molecules. These results are qualitatively similar to those obtained when the original set of peptides used to define the supermotif was subjected to the same type of analysis, and are in striking contrast with the level of cross-reactivity observed in the case of the previously mentioned control set of peptides binding to unrelated HLA alleles, in which only a few peptides (8%) bound to an allele other than the original restricting element. From the set of peptides used to validate the A3-like supermotif, 10 additional peptides binding with high or intermediate affinity to at least four of the five A3-like molecules tested were identified (Table 2B).

*High phenotypic frequencies of HLA supertypes are conserved in all major ethnic groups.* To evaluate the potential relevance of HLA supertypes in general, and of the A3-like supertype in particular, the incidence of various HLA class I alleles or antigens was examined. To date, most of the HLA-A and -B population data that are available are based on serologic typing. These data do not have resolution at the level of alleles as defined by DNA sequences, and thus do not distinguish between subtypes. However, comparison of the peptide-binding specificities of subtypes, either through peptide-binding studies (del Guercio, et al., J Immunol 154:685 (1995); Tanigaki, et al., Hum Immunol 39:155 (1994)), pool sequencing analysis (Fleischer, et al., Tissue Antigens 44:311 (1994); Rötzschke, et al., Eur J Immunol 22:2453 (1992)), or analysis of pocket structure based on primary sequence, suggest that in most instances subtypes will have very similar, if not identical, peptide main anchor specificities. Thus in the following analysis, if population data at the DNA subtype level were not available, but either binding data, published motifs, or sequence analysis suggested that subtypes will have overlapping peptide binding specificities, a one-to-one correspondence between subtypes allele and the serologically defined anti-gens was assumed.

When the incidence of the various A3-like alleles or antigens in different ethnic backgrounds was examined, it became apparent that while the frequency of each individual allele or antigen can vary drastically between ethnic groups (Imanishi, et al., Proceedings of the Eleventh International Histocompatibility Workshop and Conference, Vol. 1, Tokyo, Oxford University Press (1992)), the cumulative frequency of the five A3-like alleles is remarkably constant (in the 37% to 53% range). For example, A3 is common in Caucasians, North American blacks, and Hispanics, but almost absent in Japanese. Conversely, A31 is frequent in Japanese but rare in Caucasians and North American blacks. By contrast, in each of the five populations examined, the A3-like supertype was present in at least 37%, and as high as 53%, of the individuals.

In this context it is also interesting to note that the existence an A3-like supertype is not an isolated incident, as we have also recently reported the existence of A2-like (del Guercio, et al., J Immunol 154:685 (1995)) and B7-like (Sidney, et al., J Immunol 154:247 (1995)) supertypes.' Each of these additional supertypes is also very prominent, with remarkably constant cumulative frequencies (in the 40% to 60% range) amongst different ethnic backgrounds. In fact, it can be easily calculated that at the gene level at least one half of the total copies of HLA-A or -B genes in existence today appear to belong to one or another of these three HLA supertypes.

### DISCUSSION

The data presented here demonstrate that products from at least five different HLA alleles (A3, A11, A31, A*3301, and A*6801), and likely at least three others (A*3401, A*6601, and A*7401) predicted on the basis of pocket analysis, can be grouped into a single functional A3-like supertype. This determination was made on the basis of a number of observations. As a group, these molecules (a) share certain key structural features within their peptide-binding regions, (b) have similar preferences for their primary anchor residues, and (c) share largely overlapping binding repertoires. By examining the binding activity of a large panel of peptides beating anchor residues preferred by these allelic molecules, an A3-like supermotif was also defined. This supermotif, which is based on a detailed map of the secondary anchor requirements of each of the A3-like supertype molecules, allows for the efficient prediction of A3-like degenerate binding peptides. Finally, it was shown that the A3-like supertype, and supertypes in general, are represented with remarkably high phenotypic frequencies in all major ethnic groups. As such, HLA class I supertypes based on peptide-binding specificities represent a functional alternative to serologic and phylogenetic classification for understanding the relationships between HLA class I molecules.

Besides their use for the generation of the A3-like supermotif, the individual secondary anchor maps disclosed in this study represent in themselves a significant contribution to the understanding of peptide binding to class I molecules. Because these maps were derived using peptides of homogeneous size, the preference determinations at each of the secondary positions may be more accurate than those derived from the sequencing of pools of naturally processed peptides. Also, the motifs defined herein allow the determination of residues which have deleterious effects on peptide binding.

Barber and co-workers (Barber, et al., Curr Biol 5:179 (1995)) have demonstrated that peptides could be recognized in the context of two molecules we have included in the HLA-B7-like supertype, and two other peptides have been reported as being recognized in the context of more than one A3-like allele (Missale, et al., J Exp Med 177:751 (1993); Koenig, et al., J Immunol 145:127 (1990); Culmann, et al., J Immunol 146:1560 (1991)) (see Table 4). Using a method for in vitro induction of primary CTLs (Wentworth, et al., Mol Immunol 32:603 (1995)) we observed several instances in which peptides can be recognized in the context of both A3 and A11. We tested the A3-like supertype restricted epitopes for binding capacity to A3-like supertype molecules, and noted relatively high levels of degeneracy. Of the seven epitopes listed in Table 4, only one was a nonamer that could be analyzed for the supermotif proposed in Fig. 2A (future studies will be aimed at extending the supermotif to peptides longer than nine-mers). This peptide was supermotif positive, and bound three of five A3-like molecules. Nonetheless, it is important that each of the epitopes conformed to the A3-like supertype primary anchor specificities.

Comparison of the supertype classifications we have proposed on the basis of peptide binding with the classification of HLA-A alleles on the basis of DNA sequence (and serologic reactivity) relationships (Ishikawa, et al., Hum Immonol 39:220 (1994); Firgaira, et al., Immunogenetics 40:445 (1994); Karo, et al., J Immunol 143:3371 (1989)) reveals both similarities and differences. For example, HLA-A3 and A11 appear to be closely related and derived from a common ancestral gene (48-50). A31 and A33, however, derive from the ancient lineage comprising the A2/A10/A19 groups, which is different from the lineage of A3 and A11. Finally, HLA-A*6901 belongs to the A28 HLA evolutionary group [Fernandez-Viña, et al., Hum Immunol 33:163 (1992); Ishikawa, et al., Hum Immonol 39:220 (1994); Lawlor, et al., Annu Rev Immunol 8:23 (1990)], which also contains the HLA-A*6802 and -A*6901 alleles. Yet, on the basis of their peptide-binding specificity, HLA A*6801 is a member of the A3-like supertype, whereas A*6802 and A*6901 have been demonstrated to belong to the A2-like supertype [del Guercio, et al., J Immunol 154:685 (1995)]. Thus, based on the available phylogenetic tree of HLA alleles [Ishikawa, et al., Hum Immonol 39:220 (1994); Firgaira, et al., Immunogenetics 40:445 (1994); Karo, et al., J Immunol 143:3371 (1989)], A3-like alleles are found in both of the ancient HLA lineages: A1/A9 which includes A3 and A11, and A2/A10/A19 which includes A31, A33, and A*6801. If the existence of the HLA-A3-like supertype is reflective of common ancestry, then the A3-like motif might in fact represent primeval human HLA class I peptide-binding specificity, and other specificities may represent adaptations to changing pathogenic environments.

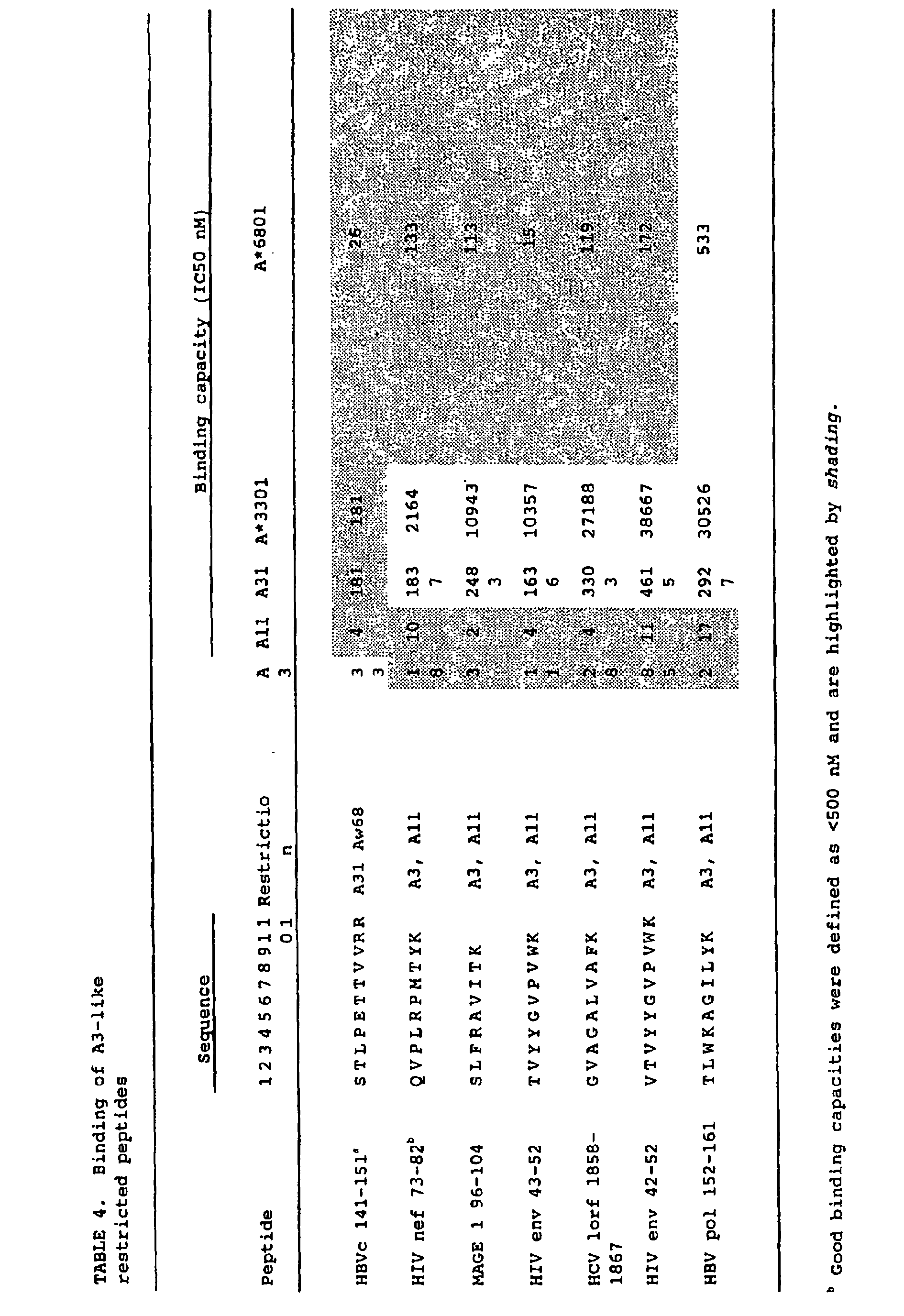

These observations, however, also raise the intriguing possibility that there might exist, at the population level, a selective advantage in maintaining high frequencies of the various HLA class I supertype alleles, generated either by common ancestry or by convergent evolution. This advantage might be related to the generation of an effective peptide-binding repertoire at the population level. This observation is evident from the fact that different molecules from the same phylogenetic family can belong to different binding supertype families. It is also possible that these phenomena might in part be related to optimal exploitation of the peptide specificity of human transporter associated with antigen processing (TAP) molecules (Androlewicz, et al., Proc Natl Acad Sci USA 90:9130 (1993); Androlewicz, et al., Immunity 1:7 (1994); van Endert, et al., Immunity 1:491 (1994); Heemels, et al., Immunity 1:775 (1994); Momburg, et al., Curr Opin Immunol 6:32 (1994); Neefjes, et al., Science 261:769 (1993)) which have been shown to preferentially transport peptides with certain sequence features such as hydrophobic, aromatic, or positively charged C-termini. Recent studies, performed by van Endert and associates, in collaboration with our own group, evaluated the relative affinities for TAP of a large collection of peptides, and have described an extended TAP binding motif. Strikingly, this motif contains many of the structural features associated with the A3-like supermotif, such as the preference for aromatic residues at positions 3 and 7 of nonamer peptides and the absence of negatively charged residues at positions 1 and 3, and P at position 1.

On the basis of the experimental evidence presented herein, it appears that, in addition to classification based on serology or phylogenetic relationships, HLA class I alleles could also be (re)classified into supertypes on the basis of their ligand specificity. Three supertypes, A2-like, A3-like, and B7-like, have presently been identified, and others, such as the B44-like supertype, are suggested from the literature *(*Fleischer, et al., Tissue Antigens 44:311 (1994); Harris, et al., J Immunol 151:5966 (1993); Thorpe, et al., Immunogenetics 40:303 (1994); Falk, et al., Immunogenetics 41:162 (1994); Falk, et al., Immunogenetics 41:165 (1994)). While it remains unknown how many supertypes will be identified and how inclusive they will be, the available data demonstrate that the phenomenon of degeneracy of peptide-binding specificities, previously thought to be restricted to HLA class II (Panina-Bordignon, et al., Eur J Immunol 19:2237 (1989); O'Sullivan, et al., J Immunol 145:1799 (1990); Busch, et al., Int Immunol 2:443 (1990)), is also a feature of peptide binding to HLA class I. Whatever the reason for the occurrence of HLA class I supertypes, their potential practical relevance should be underlined. The availability of quantitative binding assays and detailed supermotifs should allow the identification of highly cross-reactive peptides. This should, in turn, allow for broad population coverage with a cocktail of a few CTL epitopes, a possibility of great significance for the potential use of peptide-based approaches to vaccine development (Vitiello, et al., J Clin Invest 95:341 (1995)).

### Example 2

### B7-like supertype binding

Previous work (Sidney, et al., J. Immunol., 154, 247-259 (1995); Hill, et al., Nature 360, 434-439 (1992); Falk, et al., Immunogenetics 38, 161-162 (1993b); Barber, et al., Curr. Biol. 5:179 (1995); Schönbach, et al. J. Immunol. 154: 5951-5958 (1995)) has indicated that a relatively large family of HLA B specificities, collectively defined as the B7-like binding supertype, is characterized by a common peptide binding motif (P in position 2, and hydrophobic or aromatic residues at the C-terminus). In this example molecular binding assays as described above are used to examine in detail the primary anchor (position 2 and C-terminus) specificities of the five most frequent B7-like alleles (B*0702, B*3501, B51, B*5301, and B*5401).

To do so, we have synthesized and tested for binding a panel of single substitution analogs of the FHV nef 84-92 peptide (sequence FPVRPQVPL). HIV nef 84-92 binds B*0702, B*3501, B51, B*5301, and B*5401 with either high (IC50 ≤50 nM) or intermediate (IC50 50-500 nM) affinity. It was found that B7-like supertype molecules have shared and rather stringent position 2 specificities. For ail five alleles, proline was the preferred residue. With only one exception (A in the case of B*3501) all of the substitutions tested at position 2 were associated with greater than 10-fold decreases in binding affinity as compared to the proline bearing of the C-terminal anchor parent peptide. By contrast, when the binding specificity was analyzed, each HLA-B type expressed a rather unique specificity pattern at the C-terminus. For example, B*0702 preferred M, F and L, while B*5101 preferred L, I, and V. Despite these differences, the overall C-terminal specificity patterns exhibited a large degree of overlap. The aliphatic residues I and V were preferred by at least four of the five molecules, and A, L, M, F, and W were preferred or tolerated in a majority of instances. Other residues, such as Y or T, were tolerated in only isolated instances, while some (e.g., K or D) were not tolerated at all.

This data regarding primary anchor specificity is in good agreement with what was disclosed in Sidney, et al., J. Immunol., 154, 247-259 (1995). Peptides capable of degenerate B7-like supertype binding should have proline in position 2 and a hydrophobic or aromatic (V, I, L, M, F, W, A) residue at their C-terminus. In formally defining the B7-like supertype primary anchor motif we have also conservatively included Y, despite its relative lack of degeneracy, because Y constitutes the dominant signal in pool sequencing analyses (Hill, et al., Nature 360, 434-439 (1992); Falk, et al., Immunogenetics 38, 161-162 (1993b), Schönbach, et al. J. Immunol. 154: 5951-5958 (1995)) of B*3501. In summary, the primary anchor motif of the B7-like supertype is defined as P at position 2, and A, I, L, M, V, F, W, and Y at the C-terminus.

### Preferred size of B7-like supertype ligands

Class I molecules usually prefer peptides between 8 and 10 residues in length (Falk, et al., Nature 351, 290-296 (1991)), although longer peptides have been shown to bind (Massale, et al., J. Exp. Med. 177:751 (1993); Chen, et al., J. Immunol. 152:2874 (1994); Collins, et al. Nature 371-626 (1994)). To determine the optimal peptide length for the B7-like supertype, panels of 8-, 9-, 10- and 11-mer peptides representing naturally occurring viral, tumor, or bacterial sequences, and each bearing the B7-like supertype primary anchor specificity described above, were synthesized and assayed for their B7-like six per type binding capacities. It was concluded that 9 residues represents the optimal peptide length for all of the B7-like molecules examined. This assessment was true both in terms of the percent of peptides of each size bound by any molecule, but also in terms of the degree of crossreactivity observed (data not shown).

### Secondary anchor motifs of B7-like alleles and the B7-like supermotif

Other residues can act as secondary anchors, thus providing supplemental binding energy to the peptides (Ruppert, et al, Cell 74:929-937 (1993); Madden, et al. Cell 75, 693-708 (1993); Saito, et al., J. Biol. Chem. 268, 21309 (1993); Sidney, et al., Hu. Immunol. 45, 79-93 (1996); Kondo, et al, J. Immunol. 155:4307-4312 (1995); Parker, et al., J. Immunol. 152, 163-175 (1994)). It has also been shown that certain residues can have negative effects on peptide binding to class I molecules (Ruppert, et al, Cell 74:929-937 (1993); Sidney, et al., Hu. Immunol., 45, 79-93 (1996); Kondo, et al, J. Immunol. 155:4307-4312 (1995), Boehncke, et al., J. Immunol. 150, 331-341 (1993)).

To develop a B7-like supermotif allowing the efficient selection of peptides with degenerate B7-like binding capacity, we sought to define secondary anchors and secondary effects involved in peptide binding to B7-like molecules using the methods described herein. The binding capacity for the five most common B7-like molecules, B*0702, B*3501, B51, B*5301, and B*5401 of a large panel of 199 nonamer peptides representing naturally occurring viral sequences containing the B7-like supertype primary anchors (proline in position 2, and A, V, I, L, M, F, and W at the C-terminus) was determined, and the data analysed. For each position the average relative binding capacity (ARBC) of peptides carrying each of the 20 amino acids was calculated and compared to the ARBC of the entire peptide set. Because of the rare occurrence of certain amino acids, residues were grouped according to individual chemical similarities as previously described (Ruppert, et al, Cell 74:929-937 (1993)). This analysis was performed separately for B*0702, B*3501, B51, B*5301, and B*5401.

It was found that the patterns of preferences and aversions, in terms of secondary anchors, exhibited by each molecule were rather unique. For example, in the panel tested, 18 peptides had positively charged residues (R, H or K) in position 1. These peptides, as a group, were very good B*0702 binders, having an ARBC of 21. For B51, however, the same peptides were relatively poor binders, with an ARBC of 0.25. However, profound similarities in preferences could also be noted. For example, peptides bearing aromatic residues (F, W, and Y) in position one were, as a group, very good binders across the set of B7-like supertype molecules, with ARBC of 4.2, 17, 16, 20, and 70 for B*0702, B*3501, B51, B*5301, and B*5401, respectively.

The values discussed above were subsequently used to derive maps of allele-specific secondary anchor requirements for each position. To do this, preferred and deleterious residues were defined as residues associated with ARBCs that were 3-fold greater than, or 3-fold less than, respectively, the overall average. These preferred and deleterious effects are summarized in Figure 3. These secondary anchor maps more clearly reveal that while each molecule exhibited its own unique secondary anchor requirements, certain features were highly conserved amongst the B7-like molecules. For example, as indicated above, aromatic residues (F, W, and Y) at position 1 were preferred by all five of the B7-like molecules. Conversely, at position 8, acidic residues (D, E) were associated with poor binding capacity for all five molecules.

Secondary effects preferred by 3 or more of the five alleles considered, but also not deleterious for any molecule, on the one hand, or secondary effects deleterious for 3 or more of the five molecules, were defined as shared. The shared features were then incorporated into an extended B7-like supermotif defining residues associated with either poor or good binding in a majority of the molecules of the B7-like supertype.

Following this logic, it would be expected that peptides bearing supermotif preferred secondary residues would exhibit a greater degree of B7-like supertype degeneracy than those which bear none, or bear deleterious residues. This assumption was tested by determining the binding crossreactivity of an independent set of peptides bearing the B7-like primary anchor specificity. As predicted, peptides which were supermotif positive (i.e., peptides with at least one supermotif preferred secondary residues, and no deleterious residues) exhibited a substantially greater degree of crossreactivity within the B7-like supertype than supermotif negative peptides (peptides with one or more supermotif deleterious residues) (data not shown).

### Implementation of the supermotif to improve the binding capacity

HLA supermotifs can be of value in predicting highly degenerate peptides, as demonstrated herein. However, most interestingly, definition of HLA supermotifs should also allow one to engineer highly crossreactive epitopes by identifying which residues within a peptide sequence which could be analoged, or "fixed", to confer upon a peptide greater degeneracy within a supertype.

To assess this possibility, six peptides which had been shown to have a high degree of degeneracy within the B7-like supertype were selected (Table 5). Each peptide already bound at least three of the five most common B7-like molecules with either high (IC50 < 50 rtM) or intermediate (IC50 50-500 nM) affinities. These peptides were analyzed in the context of both the B7-like supermotif and the allele specific secondary anchor motifs described above to determine if particular residues within their sequences could be "fixed" to further increase their binding to the B7-like supertype molecules. This assessment found that none of the particular peptides considered contained a supermotif negative residue. Three peptides (HCV core 168, MAGE 2 170, and MAGE 3 196) each had one residue which was deleterious for a single B7-like molecule (Table 5).

Next, a panel of single substituted analogs was synthesized. Some analogs contained secondary anchor substitutions which were either supermotif positive, or positive in the context of a particular allele without being deleterious for any other Other substitutions were selected on the basis of the values disclosed here. Because the preferences for the C-terminal primary anchors were unique for each allele, substitutions at this position were also considered. Thus, for example, to test if degeneracy could be increased a number of analogs were made by substituting the supermotif positive F for the native residue in position 1. Other substitutions, such as the C-terminal L for Y in HBV pol 541 were made to address poor binding of the parent peptide to B*0702 and B*5401.

When this panel was tested for its binding capacity to molecules of the B7-like supertype, the data shown in Table 5 was generated. In every case, an F substitution in position one exhibited increased binding and/or degeneracy compared to the parent sequence. For example, MAGE 2 170 bound with high affinity to B*0702, intermediate affinity to B*3501, B51, and B*5301, but only weakly to B*5401. The F1 analog of this peptide bound all five of these molecules with high affinity.

The success of substitutions aimed at specific molecules were much harder to generalize. For example, the substitution of L at the C-terminus of HBV pol 541 for the native Y was successful in conferring binding to B*0702 while increasing the binding affinity to other molecules (significantly in the cases of B51 and B*5401). In other instances, the effect observed was not as anticipated, as demonstrated by the case of HBV env 313. This peptide bound with high affinity to B*0702, B*3501, B51, and B*5301, but only weakly to B*5401. An M in 5 analog was made to increase B*5401 binding based on the observation that the aliphatic residues (L, I, V, and M) in position 5 were positive for B*5401, and relatively neutral for other molecules. As shown in Table 5, however, the significantly increased B*5401 binding capacity achieved with the M5 analog was at the expense of lowered binding to B*0702, B51, and B*5301.

While the success of individual analogs was variable, it is notable that for each case at least one analog was capable of either improving the binding affinity, or extending the degeneracy of the parent peptide. Thus, already degenerate peptides can be discretely "fixed" to improve their binding capacity and extend their degeneracy.

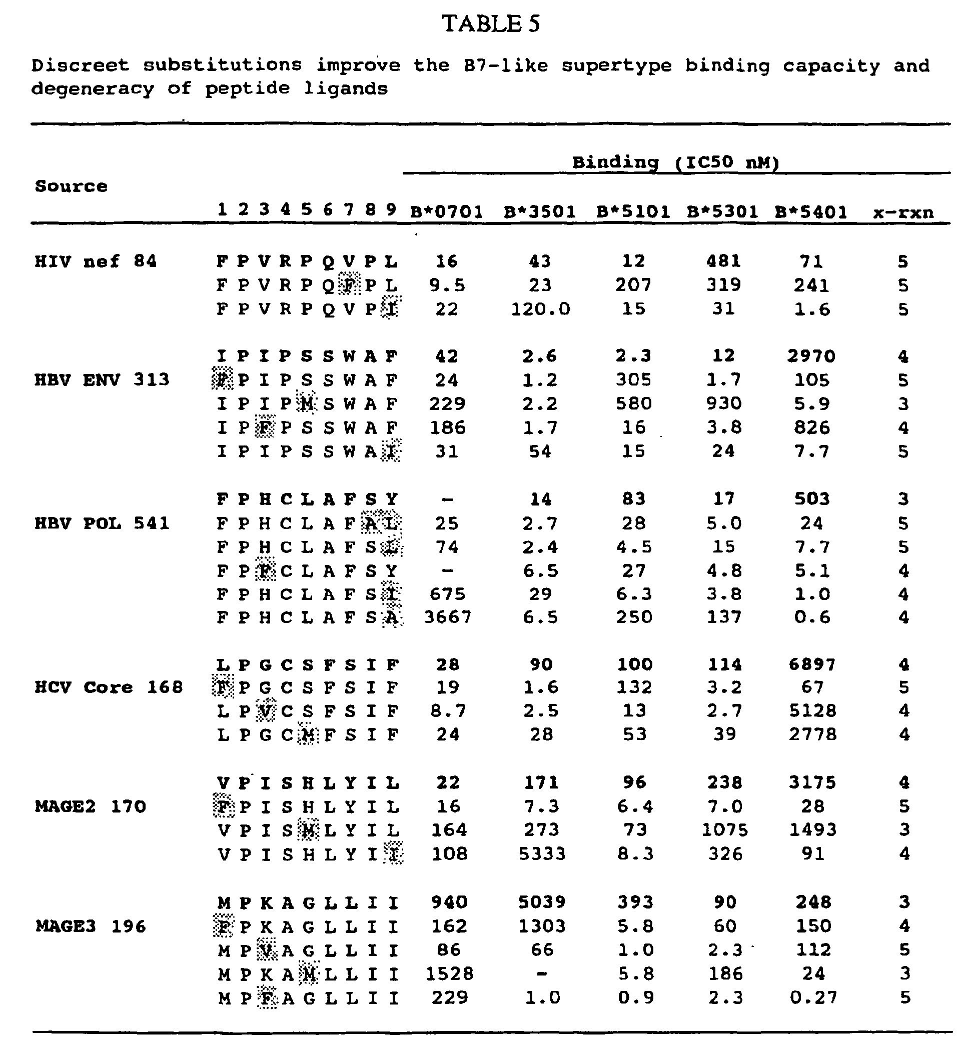

In conclusion, from the data shown herein, it is apparent that higher affinity binding peptides can be generated by substituting "negative" or neutral residues from the sequence, with either neutral or preferred residues. These peptides might also have unique immunological properties in that they, while still crossreactive with the wild type sequences, might not be subject to tolerance, deletion or suppressive mechanisms, inactivating CTL responding to the wild type sequence, and present as a result of cancer of chronic infection. The same knowledge can be utilized to generate peptides with higher degrees of crossreactivities and, thereby, more favorable population coverage.

### Example 3

### A2-like supertype binding

We have also derived further information on the structural requirements of A2.1 binding. To do this we first sought to determine the degree of permissiveness of anchor positions 2 and 9. For this purpose, a panel of analogs bearing single substitutions at either position 2 or 9 of a model poly (A) 9-mer peptide containing the previously reported A2.1 motif L in position 2 and V in position 9 (Ruppert, et al, Cell 74:929-937 (1993) was synthesized, and its binding capacity measured. Thirteen different analogs were synthesized for both anchor positions 2 and 9.

In good agreement with the previously reported A2.1 motif, the peptides carrying L or methionine (M) in position 2 were the best binders. Marked decreases in binding capacity (10- to 100-fold) were apparent even with relatively conservative substitutions such as isoleucine (I), V, alanine (A), and threonine (T). More radical changes (i.e., residues D, K, F, C, P, G, N, and S) completely abolished binding capacity. Similar results were obtained at position 9, where only conservative substitutions, such as L and I, bound within 10-fold of the unsubstituted model poly A A2.1 peptide binder. Analogs carrying A or M substitutions also bound, but less strongly (10- to 100-fold decrease). Finally, all other substitutions tested (T, C, N, F, S, G, P, and R) were associated with complete loss of A2.1 binding capacity. Thus, based on these data and in good agreement with previous studies (19-20), a "canonical" A2.1 motif could be identified as L or M in position 2 and L, V, or I in position 9.

From these results, we derived that the A2 binding of any peptide which carries a "non-canonical" (but still acceptable) residue in position 2 or 9 (or 10) (for example I, V, A or T in 2 or A, M or T in 9 or 10) could be increased by replacing it with a more "canonical" anchor. Some examples of how this can in fact be accomplished are given below.

For example, the FHV Env 2181 peptide with sequence (LWVTVYYGV) bind A2.1 with a IC50% of 12,500 nM, while the position 2 anchor substituted analog LMVTVYYGV binds with IC50% of 3.3 nM. The HBVc 18-27 naturally occurring sequence FLPSDFFPSI binds A2.1 with IC50% 22 nM, but its C-terminal anchor substituted V₁₀ variant binds A2.1 with a Kd of 2.5 nM. Finally, the HBV pol 538 peptide (YMDDVVLGA) binds A2.1 with an IC50% of 200 nM, while the V₉ variant binds with a IC50% of 5.1 nM. Other examples of fixed anchor peptides are shown in Table 6. Some of the fixed peptides were tested for their ability to induct CTL responses. For example, the HIV Env 2181 peptide and the HBV pol 721 peptides were tested in primary CTL assays (21), and found to be positive. Positive CTL recognition data exists also for the HBV_{c}18-27 and HBV pol 538 peptides.

Further experiments revealed a prominent role for non-anchor residues in determining binding capacity. The results of these analysis are also described in Ruppert *et al., supra.* According to our analysis, the frequency of a given amino acid group in A2.1 binders was divided by the frequency of nonbinders to obtain a frequency ratio. This ratio indicates whether a given group of residues occurs at a given position preferentially in binders (ratio > 1) or nonbinders (ratio < 1). To facilitate the analysis, a threshold level was set for the ratios, such that residues that had a more than 4-fold greater frequency in binders compared with nonbinders were regarded as favored or preferred residues and residues that had a more than 4-fold lower frequency in binders than in nonbinders were regarded as unfavored or deleterious residues. Following this approach, groups of residues showing prominent associations with either A2.1 binding capacity or lack thereof were identified. In general, the most detrimental effects were observed with charged amino acids. At position 1, both P and acidic (E and D) residues were infrequent in A2.1-binding peptides. At position 6, basic (H, R, and K) residues were associated with nonbinding peptides, whereas both acidic and basic residues were infrequent in good binders at positions 3 and 7. Conversely, aromatic residues were associated with high affinity binding in positions 1, 3, and 5. Furthermore, residues with OH- or SH-containing side chains, such as S, T, or C, were favored at position 4, while A was favored in position 7 and P in position 8. In conclusion, frequency analysis of different amino acid groups allowed us to define a more accurate A2.1 motif that takes into account the impact of positions other than anchor positions 2 and 9 on A2.1 binding affinity.

### Analysis of 10-mer A2.1 Ligands

The same approach described above for 9-mer peptides was also used to analyze the data obtained with a set of 10-mer peptides. At the N- and C- termini of the peptides, the pattern observed was rather similar to the one observed with 9-mers. For instance, in the 10-mer set, as in the case of the 9-mer peptides, position 1 was characterized by an increased frequency of aromatic residues in the binder set, while negative charges and P were again associated with poor binding. Again at position 3, negative charge was associated with poor binding. Interestingly, at this position, aliphatic (rather than aromatic) residues were associated with high affinity binding. At the C-termini of the peptides, certain similarities were also observed. In the 10-mer, the penultimate residue at position 9 (corresponding to position 8 in the 9-mer) was quite permissive, with only basic residues being found more frequently in nonbinders. Similar to the situation at position 7 in the 9-mer, neither positive nor negative charges were tolerated in the antepenultimate position 8 of the 10-mers. Also, position 7 did not favor positive residues in the 10-mers, as previously observed for position 6 in the 9-mers. In analogy to what was observed at position 3, the residues associated with good binding were, however, different. Aromatic and hydrophobic residues were frequent in high affinity binders at position 8 (as opposed to only A being frequent at position 7 in the 9-mers).

Finally, a rather distinctive pattern was observed in the middle of the peptide. At position 4, G was favored in high binders, while both A and positive charges were very frequent in nonbinders. P, in position 5, was completely absent in the A2.1 binders. It is noteworthy that none of the trends observed in positions 4 and 5 in the 10-mer set have any counterpart in position 4 or 5 in the 9-mer set.

In summary, a detailed motif can be generated for A2.1 10-mer peptides, following a strategy similar to the one described for 9-mer peptides above. Both important differences and striking similarities can be noted in comparing the 9-mer and 10-mer sets at these nonanchor positions.

In conclusion, from the data shown herein, it is apparent that higher affinity binding peptides can be generated by substituting "negative" or neutral residues from the sequence, with either neutral or preferred residues. These peptides might also have unique immunological properties in that they, while still crossreactive with the wild type sequences, might not be subject to tolerance, deletion or suppressive mechanisms, inactivating CTL responding to the wild type sequence, and present as a result of cancer of chronic infection.

### Binding of a peptide antigen to multiple HLA alleles allows definition of an A2-like supertype.

Direct MHC binding assays with radiolabeled peptides and HLA class I-expressing mammalian cells such as EBV-transformed B cell lines and PHA-activated blasts have been developed. Significant binding of the radiolabeled probe could be obtained if the target cells were preincubated overnight at 26°C in the presence of β2-microglobulin. Under these conditions, up to a few percent of the HLA molecules expressed by either cell type could be bound by the labeled peptides. With these assays, the degree of cross-reactivity of the A*0201-restricted hepatitis B virus core 18-27 peptide with other A2 subtypes was examined. It was determined that this peptide epitope also binds the A*0202, A*0205, and A*0206 but not A*0207 subtypes. Inhibition experiments with panels of synthetic peptide analogs underlined the similar ligand specificities of the HLA-A*0201, A*0202, and A*0205 alleles. Analysis of the polymorphic residues that help form the B and F pockets of various HLA alleles allowed prediction of binding of the hepatitis B virus core 18-27 epitope to two other HLA alleles (HLA-A*6802 and A*6901). Thus, it appears that a family of at least six different HLA-A molecules collectively defined as the A2 supertype, may share overlapping ligand specificities (aliphatic residues in position 2 and at the C termini). These results suggest that broadly crossreactive peptide epitopes can be identified and greatly enhance the prospective feasibility of peptidebased vaccination approaches.

Furthermore, these data suggest an additional use of substituted analogs. Specifically, that is to simultaneously enhance the binding affinity for several members of the A2 supertype.

An example of how this can be accomplished is given by the peptide HPV 16 EF.86-93, TLGIVCPI and its analog TLGIVXPI (where X stands for α amino butyric acid). The binding patterns of these two peptides for A2-like alleles was then tested. It was apparent that the X substitution greatly increased binding affinity for all A2-like alleles, resulting in a more useful peptide, characterized by increased binding capacity and broader crossreactivity than its original parent sequence. Subsequent experiments utilizing A2/Kb transgenic mice demonstrated that CTL induced by the X substituted peptide were fully crossreactive with the wild type sequence, and that the X peptide, as a result of its higher binding affinity, was a more potent immunogen.

### Example 4

### A24 and A1 Binding

### Secondary effects and A24 binding

We utilized a model poly (A) 9-mer peptide containing the A24 specific motif of Y in position 2 and F in position 9. It was found that in position 2 not only Y, but also F, M, and possibly W, were accepted. At the C-termini of 9 or 10 residue ligands F and W were most preferred, but also L and I were accepted. M also would be predicted to be acceptable in this position (Kubo, et al., J Immunol 152:3913 (1994)). From these results we concluded that A24 binding of any peptide which carries a non-canonical, but still acceptable residue in position 2 or 9 (or 10) (for example, F, M, W, in 2 or L, I, M, in 9 (or 10)) could be increased by replacing that residue with a more canonical anchor.

The results of further experiments describing the prominent role of non-anchor residues in determining A24 binding capacity have been described by Kondo, et al, J. Immunol. 155:4307-4312 (1995)). The overall A24 binding database was compiled, and for each position the relative average binding affinity of peptides carrying particular residues was calculated.

In the case of 9-mer peptides it was found, for example, that peptides carrying G or negatively charged residues (D, E) at position 1 tended to bind poorly, with an average affinity 10-fold lower than the average affinity of the overall set of 141 9-mer peptides, analyzed. By contrast, peptides carrying aromatic residues (F, Y, W) at position 1 bound very well with an average affinity 11.8-fold higher than the overall average.
Peptides with positively charged residues (R, K, M in position one also tended to bind well, with average affinity 4.6-fold higher than the overall average.

Negative secondary effects on A24 binding capacity were also detected at several other positions: D or E at position 3 and 6, G at position 4 and 7, positive charges (K, R, H) at position 6. A at position 8, P at position 5, and amides (Q and N) at position 5 and 8. Conversely, it was found that aromatic (Y, F, W) residues favored A24 binding when found at position 7 or 8, and small hydrogen bonding residues such as (S, T, C) had a positive effect when present at position 4.

It is apparent that every single position along the 9-mer sequence can influence A24 binding. It is also interesting that hydrophobic residues (F, W, Y, L, I, V, and M) were never associated with poor binding.

A similar analysis was also performed with the data obtained from 10-mer peptides. In analogy to what was presented in the preceding section, several secondary effects were also discerned in the case of 10 mers.

As was the case for 9-mer peptides, negative residues (D, E) in position 3 and 6 were associated with poor binding. In general, however, the map of secondary effects for 10-mers was quite distinct from that for 9-mers. For example, P, in the case of 9-mer peptides, was not associated with significantly increased binding at any position and was even associated with decreased binding at position 5. For 10-mers, P was associated with increased binding capacity when found at positions 4, 5, or 7 of 10-mer peptide ligands.

In 10-mer peptides, position 5 appears to be most important in terms of secondary effect, with (besides the already mentioned P) Y, F, and W associated with good A24 binding and R, H, and K associated with poor binding capacity. The presence of A at positions 7 and 9 and amide (Q, N) residues at positions 4 and 8 were also associated with poor binding capacity.

### Secondary effects and A1 binding

An analysis similar to that described above for A24 was also described for HLA-A*0101 molecules. Briefly, previous studies have defined two different peptide binding motifs specific for HLA-A*0101. 9 mer and 10 mer maps of secondary interactions were derived for both A*0101 submotifs. To derive such maps of secondary interactions, the relevant A*0101 binding data of peptide sets corresponding to each of the two submotifs was compiled. For each position, the relative average binding affinity of peptides carrying each particular residue was calculated. To compensate for the low occurrence of certain particular residues, and to obtain a more significant sampling, amino acids carrying chemically similar side chains were combined as by Ruppert *et al., supra.* The results obtained by this type of analysis for 9-mer peptides are shown in Figures 4a and 4b for the 2-9 and 3-9 submotifs, respectively. Diagrams illustrating the secondary effects detected by this analysis are also shown as Figures 4c and 4d (for the 2-9 and 3-9 submotifs, respectively). Increases or decreases in average affinity greater than four fold have been arbitrarily considered significant, as described above.

In general, for most positions binding capacity was affected, either negatively or positively, by the presence of particular residue types. For example, in the case of the 2-9 submotif, it was found that peptides carrying D or E position 1 bound poorly to A*0101 molecules, with an average relative binding capacity (ARBC) of 0.20. Conversely, peptides carrying aromatic residues (Y, F, or W) at the same position (position 1) bound with an affinity, on average, four-fold higher (ARBC 4-0) than the overall average binding capacity of the entire peptide set.

Inspection of the diagrams reveals some interesting features of peptide binding to A*0101. First, as noted above, the two anchors in position 2 and 3 act synergistically with each other. The affinity of peptides carrying the M, S or T anchors in position 2 is dramatically increased by the presence of D or E in 3 (and to a lesser extent by A). Conversely, the affinity of peptides carrying the D or E anchors in position 3 is dramatically increased by the presence of S, T, and M (but also other hydrophobic or short chain molecules such as L, V, I, C and A).

The degree to which peptides bearing the two submotifs differ in their interaction with the A*0101 molecule is revealed by examining other positions. Comparing the values in Figures 4a and 4b, it is clear that there are numerous examples where residues neutral in the context of one motif had positive or negative effects in the context of the other motif. At position one, for example, in the 2-9 motif G and aromatic (Y, F, and W) residues are preferred (ARBC >4.0), A and positively charged (R, H, and K) residues are relatively neutral (ARBCs between 4.0 and 0.25), and negatively charged (D and E) residues are deleterious (ARBC <0.25). In the case of peptides carrying the 3-9 submotif a different pattern is noted and, with the exception of G, which is still preferred, the preferences are shuffled. Positively charged residues in position one have a significant positive influence on peptide binding (ARBC of 8.3), negatively charged and aromatic residues are neutral (ARBCs of 1.3 and 0.61, respectively), and A is deleterious (ARBC of 0.15). Similar types of modulation are observed at each position along the motif. Overall, the shifts in secondary anchor preference from submotif to submotif is best viewed in the summary diagrams shown in Figure 3. In this context, it can be seen that, with the lone exception of the shared preference for G in position one, and excluding the position 2 and 3 co-anchors, the secondary motifs of the two A*0101 9-mer submotifs are in fact completely different. Thus, in a quantitative sense, the two 9-mer motifs have only one secondary effect out of 27 (3.7%) in common. The degree to which these A*0101 motifs differ is in striking contrast to the multiple similarities noted between the extended motifs of A24, A*0201, and A3-like molecules (20), where it was observed that between 3 and 5 (13-26%) secondary effects were shared between any two extended motifs.

### Definition of secondary anchor residues for 10-mer ligands.

In analogy to what was described in the section above for 9-mer ligands, secondary anchor residues and secondary effects were also defined for the 2-10 and 3-10 submotifs. The results of these analyses are presented in Figures 5a-d. Once again, it appeared that the anchors present in position 2 and 3 could act synergistically with each other. The presence of D, E (and to a much lesser extent A, Q and N) in position 3, in the context of the 2-10 submotif, and of hydrophobic (L, I, V, M) or short chain (S, T, C) residues in position 2, in the context of the 3-10 submotif, were associated with significant increases in average binding capacity.

Comparison of the two 10-mer motifs at positions other than 2, 3 and the C-termini indicates that, as was the case with 9-mer peptides, modulation in secondary anchor specificity occurs depending on the main anchor residues. For example, at position 7 A and S, T, and C are preferred in the 2-10 motif, but are neutral in the 3-10 motif. Conversely, G is preferred in the 3-10 motif, but is neutral in the 2-10 motif. However, it is also evident that, in contrast to the 9-mer submotifs, these differences observed in 10-mers are much less striking. In fact, the two 10-mer submotifs share a number of preferences. Y, F, and W in positions 1 and 5, A in 4, P in 7, and G in 8 had positive effects for both submotifs. Similarly, R, H, and K in 8 were deleterious in both 10-mer submotifs (Figures 5c and 5d). In total, the two 10-mer motifs shared 6 secondary effects out of 25 (24%).

## Claims

1. A method of Identifying peptides that bind to at least 3 HLA-A3 like molecules selected from the group comprising A*0301, A*1101, A*3101, A*3301 and A*6801, with a dissociation constant of less than 500 nM, the method comprising the following steps:
screening an amino acid sequence of an antigenic protein for the presence of an A3 supermotif consisting of a sequence of 9 residues from the N-terminus to the C-terminus as follows:
a first primary anchor residue at the second position selected from the group consisting of A, L, I, V, M, S and T and a second primary anchor residue at the ninth position selected from the group consisting of R and K; and
one or more secondary anchor residues selected from the group consisting of (i) Y, F, or W, at the third position, (II) Y, F, or W at the sixth position, (III) Y, F, or W at the seventh position, (Iv), P at the eighth position, and any combination of (I), (II), (III) and (Iv);
selecting one or more subsequences in the antigenic protein having the binding motif;
preparing test peptides of 8 and 11 residues comprising the selected subsequences;
determining the ability of the test peptldes to bind to the gene product;
Identifying peptides with a dissociation constant of less than 500 nM with at least 3 HLA-A3-like molecules selected from the group comprising A*0301, A*1101, A*3101, A*3301 and A*6801.

## Patentansprüche

1. Verfahren zur Bestimmung von Peptiden, welche an zumindest 3 HLA-A3-ähnliche Moleküle binden, die ausgewählt sind aus der Gruppe, welche A*0301, A*1101, A*3101, A*3301 und A*6801 umfassen, mit einer Dissoziationskonstante kleiner als 500 nM, wobei das Verfahren folgende Schritte umfaßt:
Screening einer Aminosäuresequenz eines antigenen Proteins auf das Vorhandensein eines A3-Supermotivs, das aus einer Sequenz von 9 Aminosäuren von dem N-Terminus zu dem C-Terminus besteht, wie folgt:
einer ersten primären Ankeraminosäure an der zweiten Position, die ausgewählt ist aus der Gruppe bestehend aus A, L, I, V, M, S und T, und einer zweiten primären Ankeraminosäure an der neunten Position, die ausgewählt ist aus der Gruppe bestehend aus R und K; und
einer oder mehrerer sekundärer Ankeraminosäuren, die ausgewählt sind aus der Gruppe bestehend aus (i) Y, F oder W an der dritten Position, (ii) Y, F oder W an der sechsten Position, (iii) Y, F oder W an der siebten Position, (iv) P an der achten Position, und jeder beliebigen Kombination von (i), (ii), (iii) und (iv);
Auswählen einer oder mehrerer Untersequenzen in dem antigenen Protein, welche das Bindungsmotiv aufweisen;
Herstellen von Testpeptiden mit 8 und 11 Aminosäuren, welche die ausgewählten Untersequenzen enthalten;
Bestimmen der Eignung der Testpeptide an das Genprodukt zu binden;
Ermitteln von Peptiden, die eine Dissoziationskonstante kleiner als 500 nM aufweisen und zumindest 3 HLA-A3-ähnliche Moleküle, ausgewählt aus der Gruppe, welche A*0301, A*1101, A*3101, A*3301 und A*6801 umfassen.

## Revendications

1. Méthode pour identifier des peptides qui lient au moins 3 molécules analogues de HLA-A3 choisies dans le groupe comprenant A*0301, A*1101, A*3101, A*3301 et A*6801, ayant une constante de dissociation de moins de 500 nM, cette méthode comprenant les étapes suivantes :
cribler une séquence d'acides aminés d'une protéine antigénique pour la présence d'un supermotif A3 consistant en une séquence de 9 résidus depuis le N-terminal vers le C-terminal comme suit :
un premier résidu d'ancrage primaire à la deuxième position choisi dans le groupe consistant en A, L, I, V, M, S et T et un second résidu d'ancrage primaire à la neuvième position choisie dans le groupe consistant en R et K ; et
un ou plusieurs résidus d'ancrage secondaire choisi dans le groupe consistant en (i) Y, F ou W, à la troisième position, (ii) Y, F ou W à la sixième position, (iii) Y, F ou W à la septième position, (iv), P à la huitième position, et toute combinaison de (i), (ii), (iii) et (iv) ;
choisir une ou plusieurs subséquences dans la protéine antigénique ayant le motif de liaison ;
préparer des peptides tests de 8 et 11 résidus comprenant les subséquences choisies ;
déterminer la capacité des peptides tests à se lier au produit du gène ;
identifier les peptides ayant une constante de dissociation de moins de 500 nM avec au moins 3 molécules analogues de HLA-A3 choisies dans le groupe comprenant A*0301, A*1101, A*3101, A*3301 et A*6801.
